(19) Europäisches Patentamt
European Patent Office
Office européen des brevets

(11) EP 2 114 899 B1

(12) EUROPEAN PATENT SPECIFICATION

(45) Date of publication and mention of the grant of the patent:
**22.09.2010 Bulletin 2010/38**

(21) Application number: **08702535.9**

(22) Date of filing: **28.01.2008**

(51) Int Cl.:
*C07D 239/04* (2006.01) *C07D 233/02* (2006.01)
*C11D 3/50* (2006.01) *A61Q 13/00* (2006.01)

(86) International application number:
**PCT/IB2008/050289**

(87) International publication number:
**WO 2008/093272 (07.08.2008 Gazette 2008/32)**

(54) **CONTROLLED RELEASE OF ACTIVE ALDEHYDES AND KETONES FROM EQUILIBRATED DYNAMIC MIXTURES**

KONTROLLIERTE FREISETZUNG VON AKTIVEN ALDEHYDEN UND KETONEN AUS AUSGEWOGENEN DYNAMISCHEN MISCHUNGEN

LIBÉRATION CONTRÔLÉE D'ALDÉHYDES ET DE CÉTONES ACTIFS DE MÉLANGES DYNAMIQUES ÉQUILIBRÉS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.01.2007 EP 07101370**
**21.09.2007 EP 07116890**

(43) Date of publication of application:
**11.11.2009 Bulletin 2009/46**

(73) Proprietors:
- **Firmenich S.A.**
  **1211 Geneva 8 (CH)**
- **Université de Strasbourg**
  **67081 Strasbourg (FR)**
- **Centre National de la Recherche Scientifique**
  **75794 Paris Cedex 16 (FR)**

(72) Inventors:
- **HERRMANN, Andreas**
  **CH-1255 Veyrier (CH)**
- **GODIN, Guillaume**
  **F-01550 Collonges (FR)**
- **LEHN, Jean-Marie**
  **F-67000 Strasbourg (FR)**

(74) Representative: **Carina, Riccardo Filippo**
**Firmenich SA**
**1, route des Jeunes**
**P.O. Box 239**
**1211 Genève 8 (CH)**

(56) References cited:
**WO-A-01/93823** **WO-A-2006/016248**
**WO-A-2007/079869** **US-A1- 2005 239 667**

- **JURCIK V ET AL: "Preparation of aminals in water" TETRAHEDRON, ELSEVIER SCIENCE PUBLISHERS, AMSTERDAM, NL, vol. 60, no. 14, 29 March 2004 (2004-03-29), pages 3205-3210, XP004496637 ISSN: 0040-4020 cited in the application**
- **JOHN H. BILLMAN ET AL.: "Hexahydropyrimidines." JOURNAL OF MEDICINAL CHEMISTRY., vol. 7, 1964, pages 115-117, XP002441031 USAMERICAN CHEMICAL SOCIETY. WASHINGTON.**
- **BILLMAN, JOHN H. ET AL: "Hexahydropyrimidines. VII. A study of 2-substituted 1,3-bis(2-hydroxy-3- methoxybenzyl) hexahydropyrimidines and 2-substituted 1,3-bis (3,4- dimethoxybenzyl)hexahydropyrimidines as antitumor agents" JOURNAL OF MEDICINAL CHEMISTRY , 9(3), 347-51 CODEN: JMCMAR; ISSN: 0022-2623, vol. 9, 1966, XP002441032**
- **BILLMAN, JOHN H. ET AL: "Hexahydropyrimidines. III. A study of 2-substituted 1,3-bis(p- methoxybenzyl) hexahydropyrimidines and 2-substituted 1,3-bis (p- chlorobenzyl)hexahydropyrimidines as transport molecules for tumor inhibition" JOURNAL OF MEDICINAL CHEMISTRY , 6(6), 682-3, vol. 6, 1963, pages 682-683, XP002441033**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical field

The present invention concerns a dynamic mixture obtained by combining, in the presence of water, at least one diamine derivative of formula (I), as defined further below, with at least one volatile active aldehyde or ketone. The invention's mixture is capable of releasing in a controlled and prolonged manner said active compound in the surrounding environment.

The present invention concerns also the use of said dynamic mixtures as perfuming ingredients as well as the perfuming compositions or perfumed articles comprising the invention's mixtures. A further object of the present invention is the use of said diamine derivatives as additives to prolong the perfuming effect of particular aldehydes or ketones.

### Prior art

Flavors and fragrances, but also insect attractants or repellents, are volatile molecules that can only be perceived over a limited period of time.

The perfume industry has a particular interest for compositions or additives which are capable of prolonging or enhancing the perfuming effect of a mixture of several fragrances at the same time over a certain period of time. It is particularly desirable to obtain long-lasting properties for standard perfumery raw materials which are too volatile or have a poor substantivity by themselves, or which are only deposited in a small amount onto the surface of the final application. Furthermore, some of the perfumery ingredients, especially aldehydes, are unstable and need to be protected against slow degradation prior to their use. Long-lasting perfumes are desirable for various applications, as for example fine or functional perfumery or cosmetic preparations. The washing and softening of textiles is a particular field in which there is a constant quest to enable the effect of active substances, in particular perfumes, to be effective for a certain period of time after washing, softening and drying. Indeed, many substances having odors which are particularly suitable for this type of application are known to lack tenacity on laundry, or do not remain on the laundry when rinsed, with the result that their perfuming effect is experienced only briefly and not very intensely. Given the importance of this type of application in the perfume industry, research in this field has been sustained, in particular with the aim of finding new, and more effective solutions to the aforementioned problems.

A variety of precursor compounds which release active material by a chemical reaction during or after application (using $O_2$, light, enzymes, water (pH) or temperature as the release trigger) have been described as an alternative to encapsulation systems. In general, due to their inherent instability, the precursors often decompose in the application base during storage and thus release their fragrance raw material before the desired use.

In WO 00/02991 specific amine reaction products have been prepared and isolated by reacting a primary monoamine with carbonyl compounds. However, in this system the adduct must be synthesized prior to be used and the imines which are formed are quite unstable in aqueous media. Therefore, these precursors can not easily be used in liquid applications.

In US 2005/0239667 there are disclosed systems similar to the herein above, i.e. limited to primary amines.

Similarly, WO 01/93823 reports a controlled release of fragrances from imines obtained by reaction with aromatic amines

In WO 2006/016248 it is reported a controlled release of fragrances from dynamic mixtures from a hydrazine/hydrazone equilibrium.

It is well known that carbonyl bonds can react with two amines or a diamine compound to form aminals under anhydrous conditions (see for example: S. Pawlenko and S. Lang-Fugmann, in Houben-Weyl Methoden der organischen Chemie, 1992, p. 574 as well as H. W. Wanzlick and W. Lochel, Chem. Ber. 1953, 1463). Furthermore, a new synthetic method for aminals in water was recently reported (V. Jurčík and R. Wilhelm, Tetrahedron 2004, 3205-3210). Two different secondary amines or several diamines (giving aminals with 5- to 7-membered rings) are used to synthesize these aminals.

It is important to note that, in this work, no equilibrium formation was observed and no evidence that the system may be used to control the release of volatile active aldehydes and/or ketones was given.

As a consequence of the generation of a chiral center at the 5- or 6-membered ring junction, aminals can be used to induce chirality in organocatalytic reactions (imidazolidines or hexahydropyrimidines, O. Andrey et al., Adv. Synth. Catal. 2004, 1147) or as auxiliaries in asymmetric synthesis (A. Alexakis et al., Pure & Appl. Chem. 1996, 531 and S. E. Denmark et al., J. Org. Chem. 1991, 5063). A similar application of aminals describes the kinetic resolution of aldehydes under anhydrous conditions (J. Clayden and L. W. Lai, Angew. Chem. Int. Ed. 1999, 2556 as well as J. Clayden et al., Tetrahedron 2004, 4399). The corresponding diastereoisomers formed during the reaction of diamine and aldehyde are separated by chromatography. Then the pure enantiomers are isolated after hydrolysis under acidic condition of the aminal.

Specific aminals are also known from the pharmaceutical industry or more generally from the chemical literature. However, in these cases the aminals as such are generally described as the pharmacologically active principle. They are used as simple intermediates in synthesis or disclosed as chemicals with particular properties or else as being useful for analytical purposes. None of the above-cited prior art documents reporting aminals as such suggests, or allows to reasonably expect, that the reversibility of the formation of addition products between carbonyl compounds and derivatives of formula (I) may allow to deliver said carbonyl compounds in a controlled manner or that the dynamic mixtures thus obtained can be used successfully as perfuming ingredients or even that they allow to prolong the fragrancing effect of a perfuming compound, especially in a consumer product.

Furthermore, in DE 10-2005-062175 A1 are reported aminal derivatives as classical pro-perfumes, i.e. having *"a better stability against hydrolysis"*. In this document, the principle of generating dynamic mixtures is never mentioned. The aminals reported are essentially obtained from diamines which are alkyl- or phenyl- substituted acyclic amines, which have to be prepared separately prior to their use.

We have now found that the use of totally different diamines (e.g. cyclic and/or benzylic ones) as defined further below improves the performance of volatile aldehydes in practical applications by several orders of magnitude by the in situ formation of dynamic mixtures. The increased performance is particular due to the fact that this new class of diamines provides aminals having a greater tendency to decompose by hydrolysis (in a reversible reaction) when compared to the ones disclosed in DE 10-2005-062175 A1.

To the best of our knowledge, none of the compositions of the present invention have been described or suggested for the controlled and/or improved delivery of standard (i.e. of current use) perfumery aldehydes or ketones.

## Description of the invention

We have now surprisingly found that a dynamic mixture, obtainable by combining, in the presence of water, at least one diamine derivative of formula (I) with at least one active aldehyde or ketone is a valuable ingredient capable of releasing, in a controlled and prolonged manner, said active aldehyde or ketone.

As "dynamic mixture" we mean here a composition comprising a solvent, several starting components as well as several addition products that are the results of reversible reactions between the various starting components. It is believed that said dynamic mixtures take advantage from reversible chemical reactions, in particular from the formation and dissociation by reversible condensation between the carbonyl group of the active aldehyde or ketone and the two NH moieties of the diamine derivative of formula (I). The ratio between the various starting and addition products depends on the equilibrium constant of each possible reaction between the starting components. The usefulness of said "dynamic mixture" derives from a synergistic effect between all the components.

By the term "active" we mean here that the aldehyde or ketone to which it is referred is capable of bringing a benefit or effect into its surrounding environment, and in particular a perfuming, flavoring, and/or insect repellent or attractant. Therefore, for example, said "active aldehyde or ketone" possesses at least one property which renders it useful as perfuming or flavoring ingredient, and/or as insect repellent or attractant. For a person skilled in the art, it is also evident that said active aldehydes or ketones are inherently volatile compounds.

According to all the above and below mentioned embodiments of the invention, the invention's dynamic mixture is particularly useful when the active aldehyde or ketone is a perfuming ingredient, i.e. a perfuming aldehyde or ketone. A "perfuming aldehyde or ketone" is a compound, which is of current use in the perfumery industry, i.e. a compound which is used as active ingredient in perfuming preparations or compositions in order to impart a hedonic effect. In other words, such an aldehyde or ketone, to be considered as being a perfuming one, must be recognized by a person skilled in the art of perfumery as being able to impart or modify in a positive or pleasant way the odor of a composition, and not just as having an odor. From now on we will refer to said "perfuming aldehyde or ketone" also as "perfuming compounds".

Practically, the invention is carried out exactly in the same manner, independently of the exact properties of the active aldehyde or ketone. Therefore, it is understood that, even if the invention will be further illustrated herein below with a specific reference to "perfuming compounds", the below embodiments are also applicable to other active aldehydes or ketones (i.e. it is possible to replace the expression "perfuming" with "flavoring", "insect attractant" or with "insect repellent" for instance). According to a particular embodiment of the invention, active aldehydes are preferably used.

As previously mentioned, the invention's dynamic mixture enables a controlled release of an active aldehyde or ketone, and in particular a perfuming one. Such a behavior makes the invention's dynamic mixture particularly suitable as active ingredient. Consequently, the use of an invention's dynamic mixture as active ingredient is an object of the present invention. In particular it concerns a method to confer, enhance, improve or modify the odor properties of a perfuming composition or of a perfumed article, which method comprises adding to said composition or article an effective amount of an invention's dynamic mixture.

Now, the present invention concerns a use as perfuming ingredient of a dynamic mixture, for the controlled release of active aldehydes or ketones, obtainable by reacting, in a water-containing medium,

i) at least one active aldehyde or ketone having a molecular weight comprised between 80 and 230 g/mol and being a perfuming, flavoring, insect repellent or attractant ingredient, in particular being selected from the group consisting of the $C_{5-20}$ perfuming aldehydes and the $C_{5-20}$ perfuming ketones;
with

ii) at least one derivative of formula

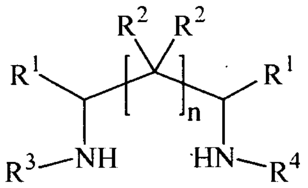
(I)

wherein:

n represents an integer varying from 0 to 3;
$R^1$ represent, independently of each others, a hydrogen atom, a phenyl group optionally substituted, or a $C_{1-18}$ alkyl or alkenyl group optionally substituted;
$R^2$ represent, independently of each others a hydrogen atom, a phenyl group optionally substituted, or a $C_{1-6}$ alkyl or alkenyl group optionally substituted; two $R^2$ or two $R^1$ or one $R^1$ and one $R^2$, taken together, may form a $C_{3-5}$ alkanediyl or alkenediyl group; and
$R^3$ and $R^4$ represent each a $C_{1-3}$ alkyl group substituted by a phenyl group optionally substituted; $R^3$ and $R^4$ or $R^3$ and the adjacent $R^1$, taken together, may form a $C_{2-4}$ alkanediyl or alkenediyl group.

Examples of possible substituents of said $R^1$, $R^2$, $R^3$ or $R^4$ comprise one, two or three groups such as $NR^6{}_2$, $(NR^6R^7{}_2)X$, $OR^7$, $SO_3$,M, $COOR^8$ or $R^7$, with $R^6$ representing a phenyl group optionally substituted by a $C_1$-$C_{10}$, or $C_1$-$C_4$, hydrocarbon group or a $C_1$ to $C_{10}$ alkyl or alkenyl group optionally comprising from 1 to 5 oxygen atoms, $R^7$ representing a hydrogen atom or a $R^6$ group, M representing a hydrogen atom or an alkali metal ion, $R^8$ representing a M group or a $R^6$ group and X representing a halogen atom or a sulphate.

The dynamic mixture is obtained by reacting one or more derivatives of formula (I) with one or more perfuming ingredients in a water-containing medium. By "water-containing medium" we mean here a dispersing medium comprising at least 10% w/w, or even 30% w/w, of water and optionally an aliphatic alcohol such as a $C_1$ to $C_3$ alcohol, for example ethanol. More preferably, said medium comprises at least 50% w/w, or even 70%, water optionally containing up to 30% of a surfactant. According to a particular embodiment of the invention, the water-containing medium may have a pH comprised between 4 and 11, and in particular between 5 and 10.

According to another particular embodiment of the invention, the preferred derivatives of formula (I) are those wherein:

n represents an integer from 0 to 2;
$R^1$ represent, independently of each others, a hydrogen atom, a phenyl group optionally substituted, or a $C_{1-4}$ alkyl group optionally substituted;
$R^2$ represent, independently of each others, a hydrogen atom, a phenyl group optionally substituted, or a $C_{1-4}$ alkyl group optionally substituted; two $R^1$ or one $R^1$ and one $R^2$ taken together, may form a $C_{3-4}$ alkanediyl or alkenediyl group; and
$R^3$ and $R^4$ represent each a $C_{1-3}$ alkyl group substituted by a phenyl group optionally substituted; $R^3$ and $R^4$ or $R^3$ and the adjacent $R^1$, taken together, may form a $C_{2-4}$ alkanediyl or alkenediyl group.

Examples of possible substituents of said $R^1$, $R^2$, $R^3$ or $R^4$ are as defined above. Alternatively, according to a further embodiment of the invention, the derivative of formula (I) is a compound of formula

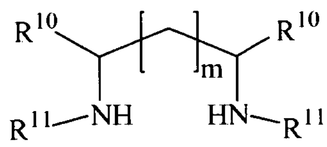

(II)

wherein m represents 0 or 1;

-$R^{10}$ represent, independently of each others, a hydrogen atom, a phenyl group optionally substituted, or a $C_{1-4}$ alkyl group optionally substituted; the two $R^{10}$, taken together, may form a $C_{3-4}$ alkanediyl or alkenediyl group; and $R^{11}$ represent, independently of each others, a $C_{1-3}$ alkyl group substituted by a phenyl group optionally substituted; two $R^{11}$ groups or one $R^{10}$ and one $R^{11}$ group, taken together, may form a $C_{2-4}$ alkanediyl or alkenediyl group.

Examples of possible substituents of said $R^{10}$ or $R^{11}$, in particular when representing a phenyl containing group, are one, two or three groups such as $NR^6_2$, $(NR^6R^7_2)X$, $OR^7$, $SO_3M$, $COOR^8$ or $R^7$ as defined above. Other substituents can be one, two or three $C_1$ to $C_{10}$ alkyl or alkenyl groups optionally comprising from 1 to 5 oxygen atoms.

According to another particular embodiment of the invention, the preferred derivatives of formula (II) are those wherein:

m represents 0 or 1;
$R^{10}$ represent, independently of each others, a hydrogen atom, a phenyl group optionally substituted, or a $C_{1-4}$ alkyl group optionally substituted; the two $R^{10}$, taken together, may form a $C_{3-4}$ alkanediyl or alkenediyl group; and
$R^{11}$ represent, independently of each others, a $C_{1-3}$ alkyl group substituted by a phenyl group optionally substituted; two $R^{11}$ or one $R^{10}$ and one $R^{11}$, taken together, may form a $C_{3-4}$ alkanediyl or alkenediyl group.

According to any one of the above embodiments of formula (II), $R^{11}$ represent, independently of each other, a $C_1$ alkyl group substituted by a phenyl group optionally substituted.

According to anyone of the above embodiments of formula (I) or (II), said diamine has preferably a molecular weight (MW) equal or above 180 g/mol (MW ≥180 g/mol), indeed said diamine is ideally odorless or has only a weak odor.

According to a particular aspect of the invention, in any of the above embodiments of the formula (I), the $R^3$ group is taken together with the adjacent $R^1$ to form an alkanediyl or alkenediyl group as defined above.

Similarly in said aspect, in any of the above embodiments of the formula (II), one $R^{10}$ and one $R^{11}$ group are taken together to form an alkanediyl or alkenediyl group as defined above.

According to a particular aspect of the invention, in any of the above embodiments of the formula (I), two $R^1$ groups, or two $R^2$ groups or one $R^2$ and one $R^1$ group, are taken together to form a group as defined above.

According to a particular aspect of the invention, in any of the above embodiments of the formula (II) the two $R^{10}$ groups are taken together to form a group as defined above.

More specifically, as non-limiting examples of diamine derivatives described in the above-mentioned embodiments, one may cite the following classes:

i) $BzNHCH_2(CH_2)_gCH_2NHBz$, wherein g is I or 0 and Bz is a substituted or non-substituted benzyl group, such as benzyl, $CH_2C_6H_4Alk$, $CH_2C_6H_4OAlk$, $CH_2C_6H_4COOAlk$ or $CH_2C_6H_4NAlk_2$ or $CH_2C_6H_4NAlk_3Cl$, with Alk being a $C_{1-4}$ alkyl group, and in particular a methyl or ethyl group;
ii) $R^{12}HN-(C_6H_{10})NHR^{12}$ wherein $R^{12}$ is a Bz group as defined above; or
iii) piperazine or 1,4-diaza-cycloheptane.

Other non-limiting examples of diamine derivatives described in the above-mentioned embodiments, comprise also the following classes:

iv) $R^{12}HNCHArCHArNHR^{12}$, wherein $R^{12}$ is a Bz group as defined above and Ar is a phenyl group; or
v) $(C_5H_9NH)CH_2NHR^{12}$ wherein $R^{12}$ is a Bz group as defined above.

As non limiting examples of diamines, one may cite the following: N,N'-dibenzylethane-1,2-diamine (N,N'-dibenzylethylenediamine), N,N'-dibenzylpropane-1,3-diamine, N,N'-dibenzylcyclohexane-1,2-diamine, N,N'-bis [4-(dimethylamino)benzyl]ethane-1,2-diamine, N,N'-bis[4-(dimethylamino)benzyl]propane-1,3-diamine, N,N'-bis(4-methoxybenzyl)ethane-1,2-diamine, N,N'-bis(4-methoxybenzyl)propane-1,3-diamine, dimethyl or diethyl 4,4'-[1,2-ethanediylbis(iminomethylene)]dibenzoate, N,N'-bis(4-ethylbenzyl)ethane-1,2-diamine, N,N'-dibenzyl-1,2-diphenylethane-1,2-diamine or N-benzyl-N-(2-piperidinylmethyl)amine.

According to some specific embodiments, the diamines N,N'-dibenzylcyclohexane-1,2-diamine or N-benzyl-N-(2-piperidinylmethyl)amine are particularly suitable.

Furthermore, the compounds of formula (I) may be in their protonated or unprotonated form. Examples of protonated forms are the one obtained by the addition of a proton to at least one of the $-NHR^3$ group to form a $-NH_2R^{3+}$ unit. Compounds of this type include in particular hydrochloride or hydrobromide derivatives of the compounds according to formula (I). Protonation and deprotonation is dependent on the pH of the medium, under highly acidic conditions for example compounds of formula (I) are expected to be in their protonated form.

Furthermore, in all the above-mentioned embodiments of the invention, the derivatives of formula (I) which are odorless, i.e. do not possess a significant odor themselves, or are even essentially non-volatile (i.e. possesses a vapor pressure of below about 150 mPa, preferably below 11 mPa, as obtained by calculation using the software EPIwin v 3.10, available at 2000 US Environmental Protection Agency) represent particularly appreciated examples, in particular for what concerns the use of the present invention in the perfumery industry.

In all the aspects of the above-described invention active compounds, and in particular the perfuming ones, are mentioned. Said active ingredients are another important element of the dynamic mixture according to the present invention.

Examples of perfuming aldehydes or ketones are available in perfumery handbooks or in the specialized literature or in the art patents, as mentioned further below.

Said perfuming compounds comprise, preferably, between 5 and 15 carbon atoms.

According to an embodiment of the invention, said perfuming aldehyde or ketone has a molecular weight comprised between 90 and 200 g/mol and can be advantageously selected from the group consisting of an enal, an enone, an aldehyde comprising the moiety $CH_2CHO$ or CHMeCHO, an aryl aldehyde or ketone (i.e. an aldehyde or ketone wherein the carbonyl functional group is directly bound to an aryl ring) and a cyclic or acyclic ketone (wherein the CO group is part or not of a cycle).

Furthermore, according to any of the embodiments mentioned above, said perfuming aldehyde or ketone is advantageously characterized by a vapor pressure above 2.0 Pa, as obtained by calculation using the software EPIwin v 3.10 (available at 2000 US Environmental Protection Agency). According to another embodiment, said vapor pressure is above 5.0, or even above 7.0 Pa.

As mentioned further above, all these embodiments apply also in the case of the active ingredient being a flavoring, insect repellent or attractant ingredient.

More specifically, as non-limiting examples of the perfuming compounds in the embodiments mentioned above, one may cite the following:

A) aldehydes of formula R"-CHO wherein R" is a linear or $\alpha$-branched alkyl group of $C_6$ to $C_{12}$, benzaldehyde. 1,3-benzodioxol-5-carboxaldehyde (heliotropine), 3-(1,3-benzodioxol-5-yl)-2-methylpropanal, 2,4-decadienal, 2-decenal, 4-decenal, 8-decenal, 9-decenal, 3-(6,6-dimethyl-bicyclo[3.1.1]hept-2-en-2-yl)propanal, 2,4-dimethyl-3-cyclohexene-1-carbaldehyde (Triplal®, origin: International Flavors & Fragrances, New York, USA), 3,5-dimethyl-3-cyclohexene-1-carbaldehyde, 1-(3,3-dimethyl-1-cyclohexyl)-1-ethanone, 5,9-dimethyl-4,8-decadienal, 2,6-dimethyl-5-heptenal (melonal), 3,7-dimethyl-2,6-octadienal (citral), 3,7-dimethyloctanal, 3,7-dimethyl-6-octenal (citronellal), (3,7-dimethyl-6-octenyl)acetaldehyde, 3-dodecenal, 4-dodecenal, 3-ethoxy-4-hydroxybenzaldehyde (ethyl vanillin), 4-ethyl benzaldehyde, 3-(2 and 4-ethylphenyl)-2,2-dimethylpropanal, 2-furancarbaldehyde (furfural), 2,4-heptadienal, 4-heptenal, 2-hexyl-3-phenyl-2-propenal (hexylcinnamic aldehyde), 2-hydroxybenzaldehyde, 7-hydroxy-3,7-dimethyloctanal (hydroxycitronellal), 4-hydroxy-3-methoxybenzaldehyde (vanillin), 4- and 3-(4-hydroxy-4-methylpentyl)-3-cyclohexene-1-carbaldehyde (Lyral®, origin: International Flavors and Fragrances, New York, USA), 4-isopropylbenzaldehyde (cuminaldehyde), 3-(4-isopropylphenyl)-2-methylpropanal, 2-(4-isopropylphenyl) propanal, 1,8-p-menthadien-7-al, (4*R*)-1-p-menthene-9-carbaldehyde (Liminal®, origin: Firmenich SA, Geneva, Switzerland), 2-and 4-methoxybenzaldehyde (anis aldehyde), 6-methoxy-2,6-dimethylheptanal (methoxymelonal), 8(9)-methoxy-tricyclo[5.2.1.0.(2,6)]decane-3(4)-carbaldehyde (Scentenal®, origin: Firmenich SA, Geneva, Switzerland), 4-methylbenzaldehyde, 2-(4-methylenecyclohexyl)propanal, 1-methyl-4-(4-methyl-3-pentenyl)-3-cyclohexen-1-carbaldehyde (Precyclemone® B, origin: International Flavors & Fragrances, New York, USA), 4-(4-methyl-3-pentenyl)-3-cyclohexene-1-carbaldehyde (Acropal®, origin: Givaudan-Roure SA., Vernier, Switzerland), (4-methylphenoxy)acetaldehyde, (4-methylphenyl)acetaldehyde, 3-methyl-5-phenylpentanal, 2-(1-methylpropyl)-1-cyclohexanone, 2,4-nonadienal, 2,6-nonadienal, 2-nonenal, 6-nonenal, 8-nonenal, 2-octenal, phenoxyacetaldehyde, phenylacetaldehyde, 3-phenylbutanal (Trifernal®, origin: Firmenich SA, Geneva, Switzerland), 3-phenylpropanal, 2-phenylpropanal (hydratropaldehyde), 3-phenyl-2-propenal (cinnamic aldehyde), 3-(4-tert-butylphenyl)-2-methylpropanal (Lilial®, origin: Givaudan-Roure SA, Vernier, Switzerland), 3-(4-tert-butylphenyl)propanal (Bourgeonal®, origin: Quest International, Naarden, Netherlands), tricyclo[5.2.1.0(2,6)]decane-4-carbaldehyde, exo-tricyclo[5.2.1.0(2,6)]decane-8exo-carbaldehyde (Vertral®, origin: Symrise, Holzminden, Germany), 2,6,6-trimethyl-bicyclo[3.1.1]heptane-3-carbaldehyde (formyl pinane), 2,4,6- and 3,5,6-trimethyl-3-cyclohexene-1-carbaldehyde, 2,2,3-trimethyl-3-cyclopentene-1-acetaldehyde (campholenic aldehyde), 2,6, 10-trimethyl-2,6,9,11 - dodecatetraenal, 2,5,6-trimethyl-4-heptenal, 3,5,5-trimethylhexanal, 2,6,10-trimethyl-9-undecenal, 2-undecenal, 10-undecenal or 9-undecenal and their mixtures such as Intreleven aldehyde (origin: International Flavors & Fragrances, New York, USA), and

B) $C_{6-11}$ ketones of formula R'-(CO)-R" wherein R' and R" are linear alkyl groups, damascenones and damascones, ionones and methyl ionones (such as Iralia® Total, origin: Firmenich SA, Geneva, Switzerland), irones, macrocyclic ketones such as, for example, cyclopentadecanone (Exaltone®) or 3-methyl-4-cyclopentadecen-1-one and 3-methyl-5-cyclopentadecen-1-one (Delta Muscenone) or 3-methyl-1-cyclopentadecanone (Muscone) all from Firmenich SA, Geneva, Switzerland, 1-(2-aminophenyl)-1-ethanone, 1-(5,5-dimethyl-1-cyclohexen-1-yl)-4-penten-1-one (Neobutenone®, origin: Firmenich SA, Geneva, Switzerland), 1-(3,3-dimethyl-1-cyclohexyl)-1-ethanone, 2,5-dimethyl-2-octene-6-one, 4,7-dimethyl-6-octene-3-one, (3,7-dimethyl-6-octenyloxy)acetaldehyde, 1-(2,4-dimethylphenyl)-1-ethanone, 4-(1,1-dimethylpropyl)-1-cyclohexanone (Orivone®, origin: International Flavors & Fragrances, New York, USA), 2,4-di-tert-butyl-1-cyclohexanone, ethyl 4-oxopentanoate, 1-(4-ethylphenyl)-1-ethanone, 2-hexyl-1-cyclopentanone, 2-hydroxy-3-methyl-2-cyclopenten-1-one, 4-(4-hydroxy-1-phenyl)-2-butanone (raspberry ketone), 1-(2- and 4-hydroxyphenyl)-1-ethanone, 4-isopropyl-2-cyclohexen-1-one, 1-(4-isopropyl-1-phenyl)-1-ethanone, 1 (6),8-p-menthadien-2-one (carvone), 4(8)-p-menthen-3-one, 1-(1-p-menthen-2-yl)-1-propanone, menthone, (1R, 4R)-8-mercapto-3-p-menthanone, 1-(4-methoxyphenyl)-1-ethanone, 7-methyl-2H,4H-1,5-benzodioxepin-3-one (Calone®, origin: C.A.L. SA, Grasse, France), 5-methyl-3-heptanone, 6-methyl-5-hepten-2-one, methyl 3-oxo-2-pentyl-1-cyclopentaneacetate (Hedione®, origin: Firmenich SA, Geneva, Switzerland), 1-(4-methylphenyl)-1-ethanone (4-methylacetophenone), 5-methyl-exo-tricyclo[6.2.1.0(2,7)]undecan-4-one, 3-methyl-4-(1,2,2-trimethylpropyl)-4-penten-2-one, 2-naphthalenyl-1-ethanone, 1-(octahydro-2,3,8,8-tetrame-2-naphthalenyl)-1-ethanone (isomeric mixture, Iso E Super®, origin: International Flavors & Fragrances, New York, USA), 3,4,5,6,6-pentamethyl-3-hepten-2-one, 2-pentyl-1-cyclopentanone (Delphone, origin: Firmenich SA, Geneva, Switzerland), 4-phenyl-2-butanone (benzylacetone), 1-phenyl-1-ethanone (acetophenone), 2- and 4-tert-butyl-1-cyclohexanone, 1-(4-tert-butylphenyl)- 1-ethanone), 2,4,4,7-tetramethyl-6-octen-3-one, 1,7,7-trimethyl-bicyclo[2.2.1]heptan-2-one (camphor), 2,6,6-trimethyl-1-cycloheptanone, 2,6,6-trimethyl-2-cyclohexene-1,4-dione, 4-(2,6,6-trimethyl-2-cyclohexen-1-yl)-2-butanone (dihydroionone), 1-(2,4,4-trimethyl-2-cyclohexen-1-yl)-2-buten-1-one, 1 -(3,5,6-trimethyl-3-cyclohcxen-1-yl)-1-ethanone, 2,2,5-trimethyl-5-pentyl-1-cyclopentanone;

wherein the underlined compounds represent, in an embodiment of the invention, particularly useful fragrance aldehydes or ketones.

As mentioned above, according to an embodiment of the invention, an active aldehyde is preferably used.

Furthermore, some of the above-mentioned compounds may also be used as perfuming, flavoring and/or insect repellent or attractant ingredients.

According to a particular embodiment of the invention said delivery systems are obtainable in a water-containing medium comprising at least 30% w/w of water, or even 50% w/w of water.

Furthermore, in all the aspects of the above-described invention the delivery systems may further comprise other amine derivatives known to generate dynamic mixtures, and in particular the hydrazine derivatives mentioned in WO 2006/016248, and/or the alkoxylamines described in a previously filed application (WO 2007/085991) or even the primary monoamine derivatives described in WO 01/93823.

The invention's dynamic mixture can be obtained by admixing together, in the presence of water, at least one compound of formula (I) and at least one perfuming compound. Although this fact is very useful in the perfumery art, where compounded perfumery ingredients are used frequently to achieve more pleasant and natural scents, it was definitively not evident that this could be operational. Indeed, the presence of several compounds capable of reacting all together (each of them with different stabilities and reactivities), could have easily led to a negative impact of the release of the individual active aldehyde or ketone thus resulting in a negative hedonic effect. This is not the case with the present invention. Therefore, a dynamic mixture obtained by reacting together at least one derivative of formula (I) with at least two, or even at least three perfuming compounds is particularly appreciated. Similarly, it is also particularly appreciated to obtain a dynamic mixture by reacting together at least one or two derivatives of formula (I) with at least two, or even at least three, perfuming compounds.

As mentioned above, the invention's dynamic mixture comprises several starting components that may react, in a reversible manner, between them to form addition products.

Now, a further aspect of the present invention concerns the dynamic mixtures themselves. Indeed, the above-mentioned dynamic mixtures are also new, and therefore represent another object of the invention. So another aspect of the present invention are the dynamic mixtures for the controlled release of active aldehydes or ketones. In particular we can mention dynamic mixtures wherein the active aldehyde or ketone is a perfuming one, as described above.

It is believed that the main components of the dynamic mixture are the free aldehyde and/or ketone, the

derivatives of formula (I) and the resulting addition products (such as the corresponding aminal derivatives). A specific example of such a mixture and equilibrium is presented in Scheme (I):

Scheme (I) : Example of an equilibrium and the species present in a dynamic mixture obtained from one specific aldehyde and one specific diamine derivative or from the corresponding aminal derivative.

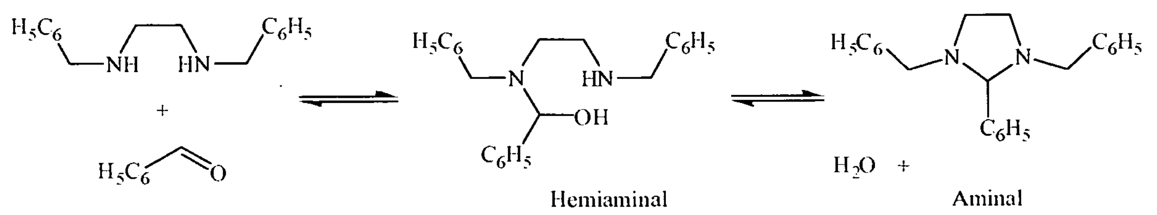

As a consequence of the fact that the reactions are reversible, a dynamic mixture can also be obtained by adding one or several aminal derivatives into water and let the mixture attain its equilibrium. However, it has to be pointed out that the time required to reach the equilibrium point can vary significantly depending on the fact that there is used, for instance, the derivative of formula (I) as starting material, as said time is believed to be dependent on various parameters such as solubilities or the basicity of the medium.

The preparation of the invention's dynamic mixture by the simple admixture of the perfuming compounds and of the derivative of formula (I) in the presence of water avoids the need of additional chemical steps such as the preparation of the corresponding aminal, and is therefore a preferred method.

Furthermore, since the aminals can also be used as precursors of the dynamic mixtures, another aspect of the invention concerns the use of said aminals as precursors of the invention's dynamic mixtures, or the use of said aminals for prolonging the perfuming effect of a perfuming aldehyde or ketone. Said aminals are of formula:

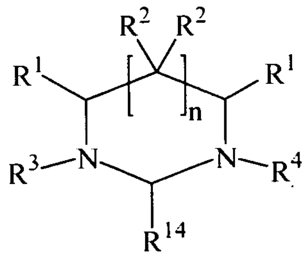

(III)

wherein n, $R^1$, $R^2$, $R^3$ and $R^4$ have the meaning as described above and $R^{14}$ is the residue derived from an active aldehyde of formula $R^{14}CHO$, said aminal being obtainable by a process comprising reacting together

- a diamine (I), as defined above, preferably having a molecular weight equal or above 180 g/mol or even above 230 g/mol; and
- an active aldehyde $R^{14}CHO$ having a molecular weight comprised between 80 and 230 g/mol and being a perfuming, flavoring, insect repellent or attractant ingredient, in particular being selected from the group consisting of the $C_{5-20}$ perfuming aldehydes and the $C_{5-20}$ perfuming ketones.

According to a particular embodiment of the invention, said active aldehyde $R^{14}$ CHO is a perfuming one. Furthermore, said active aldehyde or ketone can be a $C_{6-20}$ perfuming aldehyde or a $C_{6-20}$ perfuming ketone. According to a particular embodiment of the invention, said aminals of formula (III) are those wherein the active aldehyde $R^{14}CHO$ is one of those mentioned above. Yet according to another particular embodiment, said $R^{14}$ can be defined as $R^{17}$ herein below.

Furthermore, since some of the above aminals are also new compounds, another aspect of the invention concerns said aminals as such. Said new aminals according to the invention are of formula

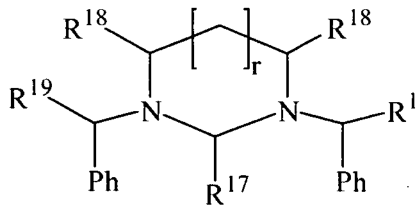

(IV)

wherein r represents 0 or 1;

$R^{19}$ represent, independently of each others, a hydrogen atom or a methyl or ethyl group;
$R^{18}$ represent, independently for each others, a hydrogen atom, a phenyl group optionally substituted by one or two OH or $C_1$-$C_4$ alkyl or alkoxyl groups, or a $C_{1-4}$ alkyl group; two $R^{18}$, taken together, may form a $C_{3-4}$ alkanediyl or alkenediyl group;
Ph represent, independently for each others, a phenyl group optionally substituted by one or two $NR^{20}_2$, $(NR^{20}_3)X$, $OR^{20}$ , $SO_3M$, $COOR^{20}$ or $R^{20}$, with $R^{20}$ representing a $C_1$ to $C_3$ or $C_4$ alkyl group or a hydrogen atom, M representing a hydrogen atom or an alkali metal ion, and X representing a halogen atom or a sulphate; and
$R^{17}$ is the residue of an active aldehyde $R^{17}CHO$ having a molecular weight comprised between 80 and 230 g/mol and being a perfuming, flavoring, insect repellent or attractant ingredient, and wherein $R^{17}$ represents a $C_6$-$C_{14}$ alkyl, alkenyl or alkadienyl group optionally substituted by an OH or an $OR^{15}$ group, or a $C_{1-3}$ alkyl or alkenyl group substituted by a phenyl group optionally substituted by one, two or three OH, $R^{15}$ or $OR^{15}$ groups, $R^{15}$ being an acetyl or a $C_1$-$C_4$ alkyl or alkenyl group;

provided that if Ph is substituted with OH or OMe groups and $R^{18}$ and $R^{19}$ are hydrogen atoms, then said $R^{17}$ represents

- a $C_7$-$C_{14}$ alkyl group or a $C_6$-$C_{14}$ alkenyl, alkadienyl group,
- a $C_{1-3}$ alkyl group substituted by a phenyl group substituted by one, two or three OH, $R^{15}$ or $OR^{15}$ groups,
- $C_{2-3}$ alkyl group substituted by a phenyl group or
- a $C_{2-3}$ alkenyl group substituted by a phenyl group substituted by one, two or three OH, $R^{15}$ or $OR^{15}$ groups,

$R^{15}$ being a $C_1$-$C_4$ alkyl or alkenyl group;
and provided that 1,2,3-tribenzyl-imidazolidine, 1,3-dibenzyl-2-styryl-imidazolidine, 1,3-dibenzyl-2-hexyl-imidazolidine and 1,3-bis(4-dimethylaminobenzyl)-2-styryl-imidazolidine are excluded.

According to a particular embodiment of the invention, said active aldehyde $R^{17}CHO$ is a perfuming one. Furthermore, said active aldehyde or ketone can be a $C_{6-20}$ perfuming aldehyde or a $C_{6-20}$ perfuming ketone. According to a particular embodiment of the invention, said aminals of formula (IV) are those wherein the active aldehyde $R^{17}CHO$ is one of the mentioned above.

According to a particular aspect of the invention, in any of the above embodiments of the formula (IV) the two $R^{18}$ groups are taken together to form a group as defined above.

Due to its nature, the invention's dynamic mixture circumvents the problem of product instability observed with prior art precursors, by the fact that a dynamic equilibrium is spontaneously set up between these compounds. This instability problem is avoided in a way significantly different from the one described in the prior art (e.g. in DE 10-2005-062175 A1) where it is always mentioned that it is preferable to increases as much as possible the degradation of the aminals against hydrolysis. In the case of the present invention, the equilibrium is stable during product storage as long as the consumer product parameters (such as concentration, temperature, pH or humidity, the presence of surfactant etc.) are kept constant. At a given set of parameters, the time required to reach the equilibrium state mainly depends on the kinetic rate constant of the slowest step involved in the formation of the products of the equilibrium.

The invention's dynamic mixture is furthermore able to stabilize active aldehydes and ketones, against degradation, in aqueous media by reversibly forming an addition product between a compound of formula (I) and the active aldehyde or ketone and thus reversibly protect the carbonyl function as an aminal function, for example of formula (III). The spontaneous reversible formation of a high amount of aminals in the dynamic mixture is thus expected to stabilize the carbonyl functionality of the active aldehyde or ketone to a large extent.

As mentioned above, the dynamic mixture of the invention comprises various components. It is believed that, once the dynamic mixture is deposited on a surface, the free perfuming aldehydes or ketones start to evaporate, diffusing in the surrounding environment their typical scent. Said evaporation perturbs the chemical equilibrium and the various addition products start to decompose so as to restore the equilibrium. The consequence of such re-equilibration is the

regeneration of free perfuming aldehydes or ketones, thus maintaining their concentration relatively constant over time and avoiding a too rapid evaporation.

Now, it has been observed that the various physical or thermodynamic properties of the dynamic mixture, e.g. its deposition on a surface or the amount of addition products formed, can be influenced by the chemical nature of the perfuming compounds or of the derivatives of formula (I). Another way to influence the above-mentioned properties is to modify the molar ratio between said perfuming compounds and the derivatives of formula (I). For instance, the lower the molar ratio between perfuming compounds and derivatives of formula (I), the longer takes the evaporation of all the perfuming compounds. The presence of other ingredients (such as surfactants, emulsifiers, gelators or others) typically used in the final consumer product formulation may also influence the above-mentioned properties.

Therefore, by varying the chemical structure of the mixture's constituents and their ratio, it is possible to fine-tune the release properties of the invention's dynamic mixture, so as to adapt its behavior to the specific requirement of the targeted consumer product.

According to the final application, a broad range for the speed of evaporation of the perfuming compound may be desirable.

The ratio between the total molar amount of perfuming aldehyde and/or ketone and the total molar amount of the compound of formula (I) can be comprised between 1:2 and 50:1, preferably between 1:1 and 10:1.

The amount of free active aldehyde or ketone present in the equilibrated dynamic mixture is comprised between 1 and 97%, preferably between 5 and 95% or even more preferably between 25 and 90%.

Another advantage of the invention resides in the fact that it is possible to fine-tune the thermodynamic behavior of the dynamic mixture by selecting the nature of the $R^1$, $R^3$ and $R^4$ groups. It is therefore conceivable to design dynamic mixtures comprising, for instance, a derivative of formula (I) which allows a fast release of a specific active aldehyde (which will be perceivable at the beginning of the consumer use only) and a second derivative of formula (I) which allows a release of the same specific aldehyde, or of another, a very slow release (which will be perceivable even after an important delay from the direct consumer use).

Moreover, another object of the present invention concerns also a composition comprising the invention's dynamic mixture. This concerns also in particular a perfuming composition comprising:

i) as perfuming ingredient, a dynamic mixture as defined above;
ii) at least one ingredient selected from the group consisting of a perfumery carrier and a perfumery base; and
iii) optionally at least one perfumery adjuvant.

Preferably, in said perfuming composition the perfumery carrier, perfumery base and perfumery adjuvant have a total molar amount of aldehydes or ketones which is equal to or higher than the molar amount of derivatives of formula (I) of the dynamic mixture.

By "perfumery carrier" we mean here a material which is practically neutral from a perfumery point of view, i.e. that does not significantly alter the organoleptic properties of perfuming ingredients. Said carrier may be a liquid. As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples solvents such as dipropyleneglycol, diethyl phthalate, isopropyl myristate, benzyl benzoate, 2-(2-ethoxyethoxy)-1-ethanol or ethyl citrate, which are the ones most commonly used.

By "perfumery base" we mean here a composition comprising at least one perfuming co-ingredient. Said perfuming co-ingredient is not an aldehyde or ketone as defined above for the dynamic mixture. Moreover, by "perfuming co-ingredient" it is meant here a compound, which is used in perfuming preparation or composition to impart a hedonic effect. In other words such a co-ingredient, to be considered as being a perfuming one, must be recognized by a person skilled in the art as being able to impart or modify in a positive or pleasant way the odor of a composition, and not just as having an odor.

The nature and type of the perfuming co-ingredients present in the base do not warrant a more detailed description here, which in any case would not be exhaustive, the skilled person being able to select them on the basis of its general knowledge and according to intended use or application and the desired organoleptic effect. In general terms, these perfuming co-ingredients belong to chemical classes as varied as alcohols, esters, lactones, ethers, acetates, nitriles, terpene hydrocarbons, nitrogenous or sulphurous heterocyclic compounds and essential oils, and said perfuming co-ingredients can be of natural or synthetic origin. A further class of perfuming co-ingredients can be the aldehydes or ketones which do not react with the diamine derivative present in the dynamic mixture.

Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds.

For the compositions which comprise both a perfumery carrier and a perfumery base, other suitable perfumery carriers, than those previously specified, can be also ethanol, water/ethanol mixtures, limonene or other terpenes, isoparaffins such as those known under the trademark Isopar® (origin: Exxon Chemical) or glycol ethers and glycol ether esters such as those known under the trademark Dowanol® (origin: Dow Chemical Company).

By "perfumery adjuvant" we mean here an ingredient capable of imparting additional added benefit such as a color, a particular light resistance, chemical stability (e.g. antioxidants) and others. A detailed description of the nature and type of adjuvant commonly used in perfuming bases cannot be exhaustive, but it has to be mentioned that said ingredients are well known to a person skilled in the art.

An invention's composition consisting of an invention's dynamic mixture and at least one perfumery carrier represents a particular embodiment of the invention as well as a perfuming composition comprising an invention's dynamic mixture, at least one perfumery carrier, at least one perfumery base, and optionally at least one perfumery adjuvant.

As anticipated above, the invention's dynamic mixtures or compositions can be advantageously used for bringing a benefit to consumer products, such as its perfuming. Indeed, said mixture possesses several other properties that make it particularly suitable for this purpose. Consequently, a consumer article comprising the invention's dynamic mixture is also an object of the present invention.

Indeed, and for example, another advantage of the invention's mixture is an improved deposition on a surface of the perfuming aldehydes or ketones compared to those of the pure aldehydes or ketones as such.

All the above-mentioned properties, i.e. improved substantivity, prolonged time of evaporation, improved stability over aggressive agents, and improved deposition, are very important for a perfuming composition. Indeed, when said compositions are intended for use in fine perfumery, the invention's mixture may allow the creation of new perfuming effects which are otherwise difficult to be achieved, such as a fresh green note being present over several hours. In the case of perfuming compositions intended for the functional perfumery, the above-mentioned properties are also very important. For example, perfuming ingredients present as such in washing compositions which have generally little staying-power on a surface are consequently often eliminated, for example in the rinsing water or upon drying of said surface. This problem can be solved by using the invention's dynamic mixture, which possesses an improved stability over storage and substantivity on surfaces, such as textiles or hair.

Therefore, the mixtures according to the invention, owing to a lower and more uniform evaporation per unit of time, resulting in a controlled release of odoriferous molecules, can be incorporated in any application requiring the effect of prolonged liberation of an odoriferous component as defined hereinabove and furthermore can impart a fragrance and a freshness to a treated surface which will last well beyond the rinsing and/or drying processes. Suitable surfaces are, in particular, textiles, hard surfaces, hair and skin.

Consequently, the invention concerns also in particular consumer article in the form of a perfumed article comprising:

i) as perfuming ingredient, a dynamic mixture as defined above; and
ii) a liquid consumer product base;

is also an object of the present invention.

Preferably, in perfumed articles the liquid consumer product base has a total molar amount of aldehydes and/or ketones which is equal to or higher than the molar amount of derivatives of formula (I) of the dynamic mixture.

For the sake of clarity, it has to be mentioned that, by "liquid consumer product base" we mean here a consumer product which is compatible with a perfume or perfuming ingredients and which is not a solid, e.g. a more or less viscous solution, a suspension, an emulsion, a gel or a cream. In other words, a perfumed article according to the invention comprises the functional formulation, as well as optionally additional benefit agents, corresponding to a consumer product, e.g. a conditioner, a softener or an air freshener, and an olfactively effective amount of an invention's dynamic mixture.

The nature and type of the constituents of the liquid consumer product base do not warrant a more detailed description here, which in any case would not be exhaustive, the skilled person being able to select them on the basis of its general knowledge and according to the nature and the desired effect of said article.

Suitable consumer products comprise liquid detergents and fabric softeners as well as all the other articles common in perfumery, namely perfumes, colognes or after-shave lotions, perfumed liquid soaps, shower or bath mousses, oils or gels, hygiene products or hair care products such as shampoos or hair sprays, body-care products, liquid based deodorants or antiperspirants, air fresheners comprising a liquid perfuming ingredient and also cosmetic preparations. As "detergents" are intended consumers products bases such as detergent compositions or cleaning products for washing up or for cleaning various surfaces, e.g. intended for textile, dish or hard-surface treatment, whether they are intended for domestic or industrial use. Other perfumed articles are fabric refreshers, ironing waters, papers, wipes or bleaches.

Preferred consumer products are perfumes, air fresheners, deodorants or antiperspirants, cosmetic preparations, ironing waters, softener bases, fabric refreshers or hair sprays.

Even more preferred consumer products are perfumes, softener bases or fabric refreshers, liquid based deodorants or antiperspirants or air fresheners comprising a liquid perfuming ingredient.

Softener bases or air fresheners comprising a liquid perfuming ingredient are particularly preferred.

According to an embodiment of the invention, it is also possible to have a perfumed article comprising:

i)

- a derivative of formula (I), as above described, and/or at least one aminal obtainable from a derivative of formula (I) and an active aldehyde or ketone as above defined; and a perfume or perfuming composition containing at least one perfuming aldehyde or ketone having a molecular weight comprised between 80 and 230 g/mol; or
- at least one aminal obtainable from a derivative of formula (I) and an active aldehyde or ketone as above defined; and

ii) a solid consumer product base intended to be used in the presence of water.

In such a case, the invention's dynamic mixture will be formed once the consumer article is used by the consumer, since water will be present. Examples of such solid consumer product bases intended to be used in the presence of water include powder detergents or "ready to use" powdered air fresheners. In particular, the aminals cited above can be one of formula (III).

Typical examples of fabric detergents or softener compositions into which the compounds of the invention can be incorporated are described in Ullman's Encyclopedia of Industrial Chemistry, vol. A8, pages 315-448 (1987) and vol. A25, pages 747-817 (1994); Flick, Advanced Cleaning Product Formulations, Noye Publication, Park Ridge, New Jersey (1989); Showell, in Surfactant Science Series, vol. 71: Powdered Detergents, Marcel Dekker, New York (1988); Proceedings of the World Conference on Detergents (4th, 1998, Montreux, Switzerland), AOCS print.

Some of the above-mentioned articles may represent an aggressive medium for the invention's compounds, so that it may be necessary to protect the latter from premature decomposition, for example by encapsulation.

The proportions in which the dynamic mixture according to the invention can be incorporated into the various aforementioned articles or compositions vary within a wide range of values. These values are dependent on the nature of the article or product to be perfumed and on the desired olfactory effect as well as the nature of the co-ingredients in a given composition when the dynamic mixtures according to the invention are mixed with perfuming co-ingredients, solvents or additives commonly used in the art.

For example, typical concentrations are in the order of 0.1 % to 30 % by weight, or even more, of the invention's dynamic mixture based on the weight of the composition into which they are incorporated. Concentrations lower than these, such as in the order of 0.01% to 5% by weight, can be used when these dynamic mixtures are applied directly in the perfuming of the various consumer products mentioned hereinabove.

Another object of the present invention relates to a method for the perfuming of a surface characterized in that said surface is treated in the presence of a dynamic mixture as defined above. Suitable surfaces are, in particular, textiles, hard surfaces, hair and skin.

Moreover, an additional aspect of the present invention is a method for prolonging the perfuming effect of a perfuming aldehyde or ketone, as defined above, characterized in that at least one derivative of formula (I), as defined above, is added to a perfuming composition or perfumed article containing at least one of said aldehyde or ketone and water. In other words, it concerns the use of a derivative of formula (1), as defined above, as additive to prolong the perfuming effect of a perfuming compositions or perfumed article containing at least one perfuming compound as defined above and water.

## Examples

The invention will now be described in further detail by way of the following examples, wherein the abbreviations have the usual meaning in the art, the temperature are indicated in degrees centigrade (°C). If not stated otherwise, the NMR spectral data were recorded on a Bruker AMX 400 spectrometer in DMSO-$d_6$ at 400 MHz for $^1$H and at 100.6 MHz for $^{13}$C, the chemical displacements $\delta$ are indicated in ppm with respect to TMS as the standard, the coupling constants J are expressed in Hz. Commercially available reagents and solvents were used without further purification if not stated otherwise. Reactions were carried out in standard glassware under $N_2$.

Although specific conformations or configurations are indicated for some of the compounds, this is not meant to limit the use of these compounds to the isomers described. According to the invention, all possible conformation or configuration isomers are expected to have a similar effect.

The following diamine derivatives can be obtained from commercial sources (some of which might be sold as

their corresponding hydrochloride salts): cis/trans-1,2-diaminocyclohexane (origin: Aldrich), (1*R*,2*R*)-1,2-diaminocyclohexane (origin: Alfa Aesar), (1*R*,2*S*)-1,2-diaminocyclohexane (origin: Fluka), cis/trans-1,3-diaminocyclohexane (origin: TCI), (1*RS*,2*SR*)-1,2-diphenylethane-1,2-diamine (origin: Aldrich), 2-(aminomethyl)piperidine (origin: Wako), N,N'-dimethylethane-1,2-diamine (N,N'-dimethylethylenediamine, origin: Aldrich), N,N'-diphenylethane-1,2-diamine (N,N'-diphenylethylenediamine, origin: Fluka) and N,N'-dibenzylethane-1,2-diamine (N,N'-dibenzylethylenediamine, origin: Aldrich).

Non commercial diamines according to the invention were prepared as follows :

*Synthesis of (1R,2R)-N,N'-dibenzylcyclohexane-1,2-diamine*

Benzaldehyde (4.65 g, 43.8 mmol) was added to a solution of (1*R*,2*R*)-1,2-diaminocyclohexane (2.50 g, 21.9 mmol) in methanol (13 ml). The reaction mixture was stirred at 70°C for 6 h. Then $NaBH_4$ (2.00 g, 52.7 mmol) was added in small portions at 70°C during 30 minutes. After stirring for 3.5 hours at room temperature, the solvent was evaporated. The residue was taken up in dichloromethane and extracted with an aqueous solution of HCl (1N), the aqueous phase was separated, then NaOH (10% aqueous solution) was added to this aqueous phase to reach a pH of 10. Extraction with diethylether (3x), drying ($Na_2SO_4$) and concentrating gave 6.02 g (93%) of the desired diamine.
$^1$H-NMR: 7.35-7.24 (m, 8 H); 7.24-7.16 (m, 2 H); 3.78 (d, J = 13.3, 2 H); 3.57 (d, J = 13.3, 2 H); 2.29-2.14 (m, 4 H); 2.02 (d, J = 13.3, 2 H); 1.68-1.57 (m, 2 H); 1.18-1.08 (m, 2 H); 1.06-0.92 (m, 2 H).
$^{13}$C-NMR: 141.58 (s); 127.96 (d); 127.69 (d); 126.31 (d); 126.28 (d); 60.17 (d); 49.92 (t); 30.65 (t); 24.51. (t).
Using a similar procedure, (1*R*,2*S*)-N,N'-dibenzylcyclohexane-1,2-diamine was prepared from (1*R*,2*S*)-1,2-diaminocyclohexane and benzaldehyde,
$^1$H-NMR: 7.36-7.25 (m, 8 H); 7.25-7.16 (m, 2 H); 3.63 (d, J = 13.3, 2 H); 3.50 (d, J = 13.3, 2 H); 2.68-2.61 (m, 2 H); 1.96 (br.s, 2 H); 1.72-1.48 (m, 4 H); 1.38-1.12 (m, 4 H),
$^{13}$C-NMR: 141.43 (s); 127.96 (d); 127.85 (d); 126.33 (d); 55.30 (d); 50.33 (t); 27.38 (t); 22.01 (t),
and (cis/trans)-N,N'-dibenzylcyclohexane-1,2-diamine from cis/trans-1,2-diaminocyclohexane and benzaldehyde.

*Synthesis of (cis/trans)-N,N'-dibenzylcyclohexane-1,3-diamine*

Benzaldehyde (5.57 g, 52.5 mmol) was added to a solution of (cis/trans)-1,3-diaminocyclohexane (3.00 g, 26.3 mmol) in methanol (30 ml). The reaction mixture was stirred at 65°C for 16 hours. Then the heating was stopped and $NaBH_4$ (2.38 g, 62.9 mmol) was added in small portions (exothermic reaction). After stirring the mixture for 5 hours at room temperature, the solvent was evaporated, the residue taken up in dichloromethane and extracted with HCl (1 N). The aqueous layer was basified with NaOH (10%), extracted with ether, dried ($Na_2SO_4$) and concentrated to give 6.83 g (89%) of the desired diamine as a mixture of the cis/trans isomers in a ratio of ca. 3:1.
$^1$H-NMR (cis): 7.34-7.24 (m, 8 H); 7.22-7.15 (m, 2 H); 3.70 (s, 4 H); 2.37-2.26 (m, 2 H); 2.17-2.09 (m, 1H); 1.90-1.70 (m, 4 H); 1.69-1.60 (m, 1H); 1.10 (qt, J = 13.1, 3.3, 1H); 0.98-0.84 (m, 1H); 0.84 (q, J = 11.4, 2 H).
$^1$H-NMR (trans): 7.34-7.24 (m, 8 H); 7.22-7.15 (m, 2 H); 3.65 (s, 4 H); 3.34 (br. s, 2 H); 2.85-2.76 (m, 2 H); 1.60-1.45 (m, 6 H); 1.37-1.23 (m, 2 H).
$^{13}$C-NMR (cis): 141.47 (s); 127.91 (d); 127.72 (d); 126.21 (d); 54.56 (d); 49.92 (t); 40.05 (t); 32.71 (t); 22.55 (t).
$^{13}$C-NMR (trans): 141.53 (s); 127.91 (d); 127.78 (d); 126.21 (d); 50.65 (d); 50.18 (t); 37.08 (t); 31.51 (t); 19.29 (t).

*Synthesis of N, N'-dibenzylpropane-1,3-diamine*

Benzaldehyde (57.2 g, 0.54 mol) was added to a solution of 1,3-diaminopropane (20.0 g, 0.27 mol) in methanol (100 ml). The solution was heated to 70°C and stirred for 3 h before $NaBH_4$ (5.0 g, 0.13 mol) was added in small portions. Stirring was continued at 70°C for 30 min, then at room temperature for another 16 h. After re-heating to 60°C, more $NaBH_4$ (4.0 g, 0.11 mol) was added. The reaction mixture was stirred at 60°C for 1 h and then left cooling to room temperature. Fractional distillation (0.04 mbar, 125-130°C) yielded 51.1 g (75%) of the desired diamine.
$^1$H-NMR: 7.34-7.25 (m, 8 H); 7.22-7.16 (m, 2 H); 3.66 (s, 4 H); 2.53 (t, J = 6.8, 4 H); 1.59(q,J=7.1,2H).
$^{13}$C-NMR: 141.53 (s); 128.38 (d); 128.21 (d); 126.74 (d); 53.58 (t); 47.70 (t); 30.15 (t).

*Synthesis of N,N'-bis[4-(dimethylamino)benzyl]propane-1,3-diamine*

1,3-Diaminopropane (2.26 ml, 26.8 mmol) was added to a solution of 4-(dimethylamino)benzaldehyde (8.00 g, 53.6 mmol) and $Na_2SO_4$ (5.00 g) in dichloromethane (100 ml). The reaction was stirred at room temperature for 3 days. After evaporation of the solvent, the residue was filtered and taken up in methanol (100 ml). $NaBH_4$ (2.00 g, 52.9 mmol) was added in small portions, which resulted in a rapid increase in temperature. After 24 hours, the solvent was

evaporated, the residue taken up in dichloromethane and washed with a saturated aqueous solution of $NaHCO_3$. The organic layer was dried ($Na_2SO_4$) and concentrated to yield 9.02 g (99%) of the desired diamine.
$^1$H-NMR: 7.09 (d, J = 8.7, 4 H); 6.64 (d, J = 8.7, 4 H); 3.53 (s, 4 H); 2.84 (s, 12 H); 2.49 (t, J = 6.7, 4 H); 1.59-1.50 (m, 2 H).
$^{13}$C-NMR: 149.24 (s); 128.54 (s); 128.54 (d); 112.19 (d); 52.65 (t); 47.12 (t); 40.25 (q); 29.59 (t).

*Synthesis of N,N'-bis[4-(dimethylamino)benzyl]ethane-1,2-diamine*

1,2-Diaminoethane (1.77 ml, 26.8 mmol) was added to a solution of 4-(dimethylamino)benzaldehyde (8.00 g, 53.6 mmol) and $Na_2SO_4$ (5.00 g) in dichloromethane (100 ml). The reaction was stirred at room temperature for 3 days. After evaporation of the solvent, the residue was filtered and taken up in methanol (100 ml). $NaBH_4$ (2.00 g, 52.9 mmol) was added in small portions, which resulted in a rapid increase in temperature. After 24 hours, the solvent was evaporated, the residue taken up in dichloromethane and washed with a saturated aqueous solution of $NaHCO_3$. The organic layer was dried ($Na_2SO_4$) and concentrated to give 7.80 g (89%) of the desired diamine.
$^1$H-NMR: 7.10 (d, J = 8.7, 4 H); 6.65 (d, J = 8.7, 4 H); 3.53 (s, 4 H); 2.84 (s, 12 H); 2.54 (s, 4 H).
$^{13}$C-NMR: 149.26 (s); 128.58 (s); 128.54 (d); 112.20 (d); 52.50 (t); 48.17 (t); 40.24 (q).

*Synthesis of N,N'-bis(4-methoxybenzyl)propane-1,3-diamine*

A solution of 1,3-diaminopropane (4.00 g, 54.0 mmol) and 4-methoxybenzaldehyde (14.70 g, 108.0 mmol) in methanol (35 ml) was heated at 65°C overnight. Then the heating was stopped and $NaBH_4$ (4.90 g, 129.5 mmol) was added in small portions during 50 min, which resulted in an increase in temperature. After stirring the mixture for 5 hours at room temperature, the solvent was evaporated, the residue taken up in dichloromethane and extracted with HCl (1 N). The aqueous layer was basified with NaOH (10%), extracted with ether, dried ($Na_2SO_4$) and concentrated. Repeating the extraction of the dichloromethane solution by following the same procedure (3x) gave a total of 11.92 g (70%) of the desired diamine.
$^1$H-NMR ($CDCl_3$): 7.21 (d, J = 8.7, 4 H); 6.84 (d, J = 8.7, 4 H); 3.79 (s, 6 H); 3.70 (s, 4 H); 2.68 (t, J = 6.7, 4 H); 1.75-1.66 (m, 2 H); 1.53 (s br., 2 H).
$^{13}$C-NMR ($CDCl_3$): 158.62 (s); 132.74 (s); 129.25 (d); 113.79 (d); 55.27 (q); 53.51 (t); 47.88 (t); 30.24 (t).

*Synthesis of N,N'-bis(4-methoxybenzyl)ethane-1,2-diamine*

A solution of 1,2-diaminoethane (3.00 g, 49.9 mmol) and 4-methoxybenzaidehyde (13.62 g, 100.0 mmol) in methanol (30 ml) was heated at 65°C for 16 hours. Then the heating was stopped and $NaBH_4$ (4.54 g, 120.0 mmol) was added in small portions during 30 minutes, which resulted in an increase in temperature. After stirring the mixture for 4 h at room temperature, the solvent was evaporated, the residue taken up in dichloromethane and extracted with HCl (1 N). The aqueous layer was basified with NaOH (10%), extracted with ether, dried ($Na_2SO_4$) and concentrated. Repeating the extraction of the dichloromethane solution by following the same procedure gave a total of 12.60 g (84%) of the desired diamine.
$^1$H-NMR: 7.25-7.18 (m, 4 H); 6.90-6.82 (m, 4 H); 3.72 (s, 6 H); 3.58 (s, 4 H); 2.54 (s, 4 H).
$^{13}$C-NMR: 157.90 (s); 132.95 (s); 128.91 (d); 113.38 (d); 54.90 (q); 52.30 (t); 48.18 (t).

*Synthesis of N,N'-bis(4-ethylbenzyl)ethane-1,2-diamine*

A solution of 1,2-diaminoethane (5.00 g, 83.2 mmol) and 4-ethylbenzaldehyde (22.30 g, 166.2 mmol) in methanol (45 ml) was heated at 65°C for 16 hours. After cooling to room temperature, $NaBH_4$ (7.60 g, 200.9 mmol) was added in small portions during 35 minutes, which resulted in an increase in temperature. After stirring the mixture for 5 hours at room temperature, the solvent was evaporated, the residue taken up in dichloromethane and extracted with HCl (1 N). The aqueous layer was basified with NaOH (10%), extracted with ether, dried ($Na_2SO_4$) and concentrated. Repeating the extraction of the dichloromethane solution by following the same procedure gave a total of 19.64 g (83%) of the desired diamine.
$^1$H-NMR ($CDCl_3$): 7.21 (d, J = 8.2, 4 H); 7.14 (d, J = 7.7, 4 H); 3.73 (s, 4 H); 2.74 (s, 4 H); 2.62 (q, J = 7.7, 4 H); 1.22 (t, J = 7.4, 6 H).
$^{13}$C-NMR ($CDCl_3$): 142.85 (s); 137.81 (s); 128.13 (d); 127.85 (d); 53.70 (t); 48.85 (t); 28.53 (t); 15.64 (q).

*Synthesis of (1RS,2SR)-N,N'-dibenzyl-1,2-diphenylethane-1,2-diamine*

Benzaldehyde (0.80 g, 7.6 mmol) was added to a solution of (*1*RS,*2*SR)-1,2-diphenylethane-1,2-diamine (0.80 g, 3.8 mmol) in methanol (15 ml). The reaction mixture was stirred at 65°C for 16 hours. Then the heating was stopped

and $NaBH_4$ (0.34 g, 9.1 mmol) was added in small portions during 20 minutes (exothermic reaction). After stirring the mixture for 5 hours at room temperature, the solvent was evaporated, the residue taken up in dichloromethane and extracted with HCl (1 N). The aqueous layer was basified with NaOH (10%), extracted with ether, dried ($Na_2SO_4$) and concentrated. Plug filtration ($SiO_2$, ethyl acetate) gave 0.32 g (22%) of the desired diamine.
$^1$H-NMR ($CDCl_3$): 7.37-7.24 (m, 10 H); 7.24-7.13 (m, 6 H); 7.00-6.94 (m, 4 H); 3.75 (s, 2 H); 3.54 (d, J = 13.8, 2 H); 3.30 (d, J = 13.8, 2 H); 1.70 (br. s, 2 H).
$^{13}$C-NMR ($CDCl_3$): 140.81 (s); 140.32 (s); 128.60 (d); 128.36 (d); 128.19 (d); 127.88 (d); 127.64 (d); 126.67 (d); 67.19 (d); 50.95 (t).

*Synthesis of N-benzyl-N-(2-piperidinylmethyl)amine*

Benzaldehyde (5.11 g, 48.1 mmol) was added to a solution of 2-(aminomethyl)piperidine (5.00 g, 43.8 mmol) in methanol (50 ml). The solution was heated to 60°C and stirred for 12 h before $NaBH_4$ (2.00 g, 52.9 mmol) was added in small portions. Stirring was continued at 60°C for 1 h, then more $NaBH_4$ (2.00 g, 52.9 mmol) was added. After stirring at 60°C for 1 h, the mixture was left cooling to room temperature. Evaporation of the solvent and Kugelrohr distillation (0.09 mbar, 120°C) yielded 6.02 g (61 %) of the desired diamine.
$^1$H-NMR ($CDCl_3$): 7.36-7.28 (m, 4 H); 7.28-7.19 (m, 1H); 3.77 (d, J = 2.6, 2 H); 3.09-3.01 (m, 1H); 2.68-2.44 (m, 4 H); 1.86-1.70 (m, 3 H); 1.63-1.51 (m, 2 H); 1.48-1.25 (m, 2 H); 1.15-1.02 (m, 1H).
$^{13}$C-NMR ($CDCl_3$): 140.62 (s); 128.34 (d); 128.04 (d); 126.85 (d); 56.64 (d); 55.48 (t); 54.19 (t); 46.83 (t); 30.94 (t); 26.69 (t); 24.72 (t).

Non commercial aminal derivatives according to the invention were prepared as follows :

*Synthesis of 1,3-dimethyl-2-phenylimidazolidine (used as reference of the prior art)*

Under vigorous stirring, benzaldehyde (1.47 g, 27.7 mmol) was slowly added to a solution of N,N'-dimethylethane-1,2-diamine (1.22 g, 13.8 mmol) in water (15 ml). After 3 hours, the mixture was extracted with $CHCl_3$, the organic phase dried ($Na_2SO_4$) and concentrated to give 1.91 g (78%) of the desired aminal.
$^1$H-NMR: 7.42-7.28 (m, 5 H); 3.30-3.18 (m, 2 H); 3.22 (s, 1H); 2.54-2.42 (m, 2 H); 2.05 (s, 6 H).
$^{13}$C-NMR: 140.05 (s); 128.75 (d); 128.21 (d); 127.86 (d); 91.59 (d); 52.73 (t); 39.03 (q).

*Synthesis of 1,3-dibenzyl-2-phenylimidazolidine*

Under vigorous stirring, benzaldehyde (0.53 g, 5.0 mmol,) was slowly added to a solution of N,N'-dibenzylethane-1,2-diamine (1.20 g, 5.0 mmol) in water (7.5 ml). After ca. 5 minutes a white solid was formed. The reaction mixture was stirred for 3 hours then the residue was filtered and dried under reduced pressure to give 1.60 g (97%) of the desired aminal.
$^1$H-NMR: 7.63 (d, J = 6.7, 2 H); 7.41 (*t, J =* 7.4, 2 H); 7.38-7.30 (*m,* 1H); 7.30-7.14 (*m*, 10 H); 3.87 (s, 1H); 3.63 (d, J = 12.8, 2 H); 3.23 (d, J = 13.3, 2 H); 3.02 (dt, J = 4.6, 8.7, 2 H); 2.46 (dt, J = 4.6, 8.2, 2 H).
$^{13}$C-NMR: 140.47 (s); 138.90 (s); 129.10 (d); 128.40 (d); 128.04 (d); 128.01 (d); 126.65 (d); 87.95 (d); 55.98 (t); 50.12 (t).

*Synthesis of (±)-1,3-dibenzyl-1-(phenylpropyl)imidazolidine*

A mixture of 3-phenylbutanal (Trifernal®, 0.62 g, 4.2 mmol), N,N'-dibenzyl-1,2-ethanediamine (1.00 g, 4.2 mmol, 1 eq.) and $K_2CO_3$ in ethanol (6.2 ml) was heated to 60°C for 24 h. Then the solvent was removed under vacuum at 40°C. The residue was taken up in ether and the solvent evaporated to yield 1.28 g (83%) of the desired aminal as a mixture of diastereoisomers.
$^1$H-NMR: 7.37-7.19 (m, 12 H); 7.17-7.07 (m, 3 H); 3.85 (d, J = 13.3, 1H); 3.72 (d, J = 13.3, 1H); 3.43 (d, J = 4.6, 1H); 3.40 (d, J = 4.6, 1H); 3.21-3.16 (m, 1H); 3.06-2.95 (m, 1H); 2.84-2.71 (m, 2 H); 2.54-2.42 (m, 2 H); 1.92-1.82 (m, 2 H); 1.80-1.69 (m, 2 H); 1.15 (d, J = 7.2, 3 H).
$^{13}$C-NMR: 147.97 (s); 139.69 (s); 139.53 (s); 128.37 (d); 128.33 (d); 128.18 (d); 128.05 (d); 128.03 (d); 126.70 (d); 126.64 (d); 126.62 (d); 125.48 (d); 83.23 (d); 58.39 (t); 57.92 (t); 49.92 (t); 40.85 (t); 35.59 (d); 23.47 (q).

*Synthesis of 1,3-dibenzyl-2-phenyloctahydro-1H-benzoimidazole*

Under vigorous stirring, benzaldehyde (0.36 g, 3.4 mmol) and 0.1 ml of acetic acid were slowly added to a solution of (1*R*,2*R*)-N,N'-dibenzylcyclohexane-1,2-diamine (1.00 g, 3.4 mmol) in water (10 ml). After 24 hours, the reaction mixture was filtered, the solid was taken up in diethylether and wished with water. Drying ($Na_2SO_4$) and concentrating gave 0.65 g (44%) of the desired aminal.

$^1$H-NMR: 7.24-7.08 (m, 13 H); 7.04 (d, J = 6.7, 2 H); 4.56 (s, 1H); 3.73 (d, J = 13.8, 1H); 3.62 (d, J = 14.3, 1 H); 3.50 (d, J = 14.8, 1 H); 3.35 (s, 2 H); 3.22 (d, J = 14.8, 1 H); 2.81-2.71 (m, 1 H); 2.48-2.38 (m, 1 H); 1.74-1.56 (m, 4 H); 1.24-1.04 (m, 4 H).
$^{13}$C-NMR: 140.68 (s); 140.50 (s); 139.06 (s); 129.17 (d); 128.37 (d); 127.69 (d); 127.64 (d); 127.59 (d); 127.39 (d); 127.26 (d); 126.47 (d); 126.18 (d); 85.14 (d); 68.05 (d); 66.65 (d); 55.75 (t); 51.35 (t); 29.93 (t); 29.31 (t); 23.99 (t); 23.93 (t).

*Synthesis of 1,3-dibenzyl-2-phenylhexahydropyrimidine*

Under vigorous stirring, benzaldehyde (0.63 g, 5.9 mmol) was slowly added to a solution of N,N'-dibenzylpropane-1,3-diamine (1.50 g, 5.9 mmol) in water (10 ml). After 48 hours the reaction mixture was filtered and the solid washed with water (50 ml). Drying under reduced pressure to give 2.01 g (95%) of the desired aminal.
$^1$H-NMR ($CDCl_3$): 7.67 (*d, J = 7.2,* 2 H); 7.36 (*t, J =* 7.4, 2 H); 7.31-7.12 (*m,* 11 H); 3.61 (*d, J =* 13.3, 2 H); 3.61 (*s*, 1 H); 3.03-2.94 (*m,* 2 H); 2.85 (*d, J* = 13.3, 2 H); 2.04 (*dt, J* = 11.8, 2.0, 2 H); 1.92-1.78 (*m*, 1 H); 1.50-1:42 (*m,* 1 H).
$^{13}$C-NMR ($CDCl_3$): 141.86 (s); 139.63 (s); 129.56 (d); 128.65 (d); 128.36 (d); 128.25 (d); 128.01 (d); 126.61 (d); 89.07 (d); 58.45 (t); 51.78 (t); 24.40 (t).

Use of active aldehydes or ketones

The following examples illustrate the formation of dynamic mixtures using perfuming or flavoring ingredients as active aldehydes or ketones. However, they are also representative for the generation of dynamic mixtures according to the present invention in which the active aldehydes or ketones are useful as insect repellants or attractants. Some of the compounds described in the following examples, such as benzaldehyde, decanal, 2,4-dimethyl-3-cyclohexene-1-carbaldehyde, 3,7-dimethyl-6-octenal (citronellal), 2-furancarbaldehyde (furfural), 2-heptanone, 1,8-p-menthadien-7-al, 1-(4-methylphenyl)-1-ethanone (4-methylacetophenone), or 10-undecenal, are also known to be insect attractants or repellents (see for example: A. M. El-Sayed, The Pherobase 2005, http://www.pherobase.nef).

Example 1

Formation of an invention's dynamic mixture

The formation of the dynamic mixture was monitored by $^1$H-NMR spectroscopy in a deuterated aqueous buffer solution ($DMSO-d_6/D_2O$ 2:1 (v/v)). The aqueous part of the deuterated buffer stock solution was prepared from the following product quantities:

| | |
|---|---|
| $Na_2HPO_4$ | 0.817 g |
| $KH_2PO_4$ | 0.107 g |
| $D_2O$ | 22.10 g (= 20 ml) |

Addition of 1.0 ml of $DMSO-d_6$ to 0.5 ml of the aqueous part of the deuterated buffer stock solution gives the final reaction solution for which a pH of 6.5.-7.0 was measured (with Merck Neutralit® pH indicator paper 5.5-9.0).
To verify the formation of the same equilibrium for the formation and hydrolysis of aminal derivatives according to the present invention, 180 mM solutions of a diamine derivative, an active aldehyde or ketone and the corresponding aminal derivative, were prepared in $DMSO-d_6$, respectively. To 0.3 ml of the aqueous part of the deuterated buffer stock solution were then added in an NMR tube either 0.05 ml of the solution with the diamine derivative, 0.05 ml of the solution with the active aldehyde or ketone and 0.5 ml of $DMSO-d_6$ or, alternatively, 0.05 ml of the corresponding aminal derivative and 0.55 ml of $DMSO-d_6$, respectively. Each tube thus contains a mixture of $DMSO-d_6/D_2O$ 2:1 (v/v). The NMR tubes were sonicated for 1 hour and then left equilibrating at room temperature for 2 days before recording the $^1$H-NMR spectra of the samples. For each sample the amount of free active aldehyde or ketone with respect to the amount of the aminal derivative was determined by integration of the corresponding signals. Another NMR measurement after 4 days showed that the equilibrium did not change.
The following amounts of free active aldehydes or ketones were detected from the sample containing the diamine derivative together with an active aldehyde or ketone as compared to the reference sample containing the corresponding aminal derivative after 2 days:

| Equilibrated dynamic mixtures obtained in DMSO-$d_6$/$D_2O$ 2:1 (v/v) from | Amount of free aldehyde[a]) |
|---|---|
| N,N'-dimethylethane-1,2-diamine and benzaldehyde | 19% |
| 1,3-dimethyl-2-phenylimidazolidine | 16% |
| N,N'-dibenzylpropane-1,3-diamine and benzaldehyde | 49% |
| 1,3-dibenzyl-2-phenylhexahydropyrimidine | 45% |
| (1*R*,2*R*)-N,N'-dibenzylcyclohexane-1,2-diamine and benzaldehyde | 42% |
| 1,3-dibenzyl-2-phenyloctahydrobenzoimidazole | 54% |
| [a]) the sum of the amount of free active aldehyde (= the amount of diamine derivative) and the corresponding aminal is 100%. | |

The data show that within the experimental error (ca. 5-10%) almost the same amount of free active benzaldehyde and thus the same equilibrium is reached for a dynamic mixture obtained by reversible reaction of a diamine derivative with an active aldehyde or ketone in a water-containing medium or, alternatively, by hydrolysis of the corresponding aminal derivative. A low value, e.g below 25%, of free active aldehyde or ketone furthermore indicates an increased effect of stabilization of the compound in the aqueous medium as the labile carbonyl function is protected in the form of an aminal.

Using the same conditions, the formation of the corresponding aminal was verified after 2 days for equimolar mixtures of:

| Equilibrated dynamic mixtures obtained in DMSO-d6/$D_2O$ 2:1 (v/v) from | Amount of free aldehyde |
|---|---|
| N,N'-bis-(4-dimethylaminobenzyl)ethane- 1,2-diamine and benzaldehyde | 49% |
| N,N'-bis-(4-dimethylaminobenzyl)ethane-1,3-diamine and benzaldehyde | 52% |
| N,N'-bis(4-methoxybenzyl)propane-1,3-diamine and benzaldehyde | 34% |

Example 2

Reversibility of the equilibration of an invention's dynamic mixture

To show that the same equilibrium was obtained in both directions of the reaction and to determine the corresponding equilibrium constant, the formation and hydrolysis of the aminals according to the invention was followed by $^1H$-NMR in a deuterated aqueous buffer stock solution (THF-$d_8$/$D_2O$ 2:1 (v/v)) at different time intervals. The aqueous part of the deuterated buffer stock solution was prepared as descibed above (Example 1).

For the measurements 180 mM solutions of a diamine derivative and an active aldehyde were prepared in THF-$d_8$, respectively. Similarly, a 90 mM solution of the corresponding aminal was prepared in the same solvent. To 0.3 ml of the aqueous buffer stock solution were then added in an NMR tube either 0.05 ml of the solution with the diamine derivative and 0.05 ml of the solution with the active aldehyde and 0.50 ml of THF-$d_8$ or, alternatively, 0.10 ml of the corresponding aminal derivative and 0.50 ml of THF-$d_8$, respectively. Each tube thus contains a mixture of THF-$d_8$/$D_2O$ 2:1 (v/v). The NMR tubes were sonicated for 1 hour. $^1H$-NMR spectra of the samples were measured at different time intervals during several days. For each sample the mole fraction x of the aminal derivative was determined by integration of the corresponding $^1H$-NMR signals. The following data were obtained for the hydrolysis of 1,3-dibenzyl-2-phenylhexahydropyrimidine:

| Time [h] | 0.00 | 17.15 | 23.98 | 41.83 | 286.03 |
|---|---|---|---|---|---|
| x | 1.00 | 0.63 | 0.58 | 0.50 | 0.36 |

or the reaction of benzaldehyde and N,N'-dibenzylpropane-1,3-diamine

| Time [h] | 0.00 | 17.02 | 23.85 | 41.70 | 285.92 |
|---|---|---|---|---|---|

(continued)

| | | | | | |
|---|---|---|---|---|---|
| x | 0.00 | 0.11 | 0.17 | 0.22 | 0.36 |

The kinetics follow the general equation

$$A + B \underset{k'}{\overset{k}{\rightleftharpoons}} C$$

with *k* and *k*' being the rate constants for the forward and reverse reaction, respectively. In this case the forward reaction is second order and the reverse reaction first order (see for example: J. W. Moore, R. G. Pearson, "Kinetics and Mechanism" (3rd Ed.), John Wiley & Sons, New York, 1981, p. 284-333). By expressing the concentrations of the respective compounds as their mole fractions x, varying between 0 and 1, and with $x_e$ being the mole fraction at the equilibrium and $[A_0]$ being the concentrations of A at time $t = 0$, respectively, one obtains

$$x = \frac{x_e (e^{Qkt} - 1)}{e^{Qkt} - x_e^2} \quad \text{with} \quad Q = \frac{[A_0](1 - x_e^2)}{x_e}$$

for the forward reaction and

$$x = 1 - \frac{(1 - x_e)(e^{Qk't} - 1)}{x_e + e^{Qk't}} \quad \text{with} \quad Q = \frac{1 + x_e}{1 - x_e}$$

for the reverse reaction. The rate constant *k* (1.80 l $mol^{-1}$ $h^{-1}$) was then obtained by fitting the experimental values to the calculated ones, *k*' (0.02 l $mol^{-1}$ $h^{-1}$) as well as the equilibrium constant $K_{eq}$ (87.89) were then calculated from the relation $k = k' \cdot K_{eq}$. The data are illustrated in Figure 1. The determined value of $K_{eq}$ corresponds to the one calculated from

$$K_{eq} = \frac{x_e}{(1 - x_e)^2 [A_0]}$$

Using the same procedure for the formation and hydrolysis of 1,3-dibenzyl-2-phenylimidazolidine, $K_{eq}$ was determined to be 1200 (with $k$ = 14.78 l $mol^{-1}$ $h^{-1}$ and $k'$ = 0.01 l $mol^{-1}$ $h^{-1}$).

The data show that the reaction is reversible and that the same equilibrium was obtained for the formation and hydrolysis of the aminals according to the invention.

Example 3

Performance of a softener base comprising an invention's dynamic mixture

The use as perfuming ingredient of the present invention's mixture has been tested in a fabric softener. A fabric softener base with the following final composition has been prepared:

| | |
|---|---|
| Stepantex® VK90 (origin: Stepan) | 16.5 % by weight |
| Calcium chloride | 0.2 % by weight |
| Water | 83.3 % by weight. |

The perfuming performance, over time, of the free perfuming aldehydes/ketones and of the invention's mixtures (i.e. the free perfuming aldehydes/ketones with an diamine derivative as additive) was determined in the following experiment:

(1*R*,2*R*)-N,N'-dibenzylcyclohexane-1,2-diamine (73.4 mg, 2.46 mmol) was weighed into a small vial. Then 1.80 g of the above mentioned fabric softener base, 1 ml of a solution containing equimolar amounts (0.41 mmol) of 2-furancarbaldehyde (furfural, 39.4 mg), (*R*)-3,7-dimethyl-6-octenal (citronellal, 63.2 mg), 3-phenylbutanal (Trifernal®, 60.8 mg), 2-pentyl-1-cyclopentanone (Delphone, 63.2 mg) 10-undecenal (69.0 mg) and (±)-exo-tricyclo[5.2.1.0 (2,6)]decane-8exo-carbaldehyde (Vertral®, 67.3 mg) in 10 ml of ethanol and 1 ml of ethanol was added. Similarly, a second vial which does not contain (1*R*,2*R*)-N,N'-dibenzylcyclohexane-1,2-diamine was prepared to serve as the reference. The two samples were closed and left standing at room temperature to equilibrate. After 5 days, the samples were dispersed in a beaker with 600 ml of demineralized cold tap water, respectively. One cotton towel (EMPA cotton test cloth Nr. 221, origin: Eidgenössische Materialprüfanstalt (EMPA), pre-washed with an unperfumed detergent powder and cut to ca. 12 x 12 cm sheets) was added to each beaker and agitated manually for 3 minutes, left standing for 2 minutes, then wrung out by hand and weighed to obtain a constant quantity of residual water. The two towels were left drying overnight and analyzed the next day. Each towel was put into a headspace sampling cell (160 ml) thermostated at 25°C and exposed to a constant air flow of ca. 200 ml/min. The air was filtered through active charcoal and aspirated through a saturated solution of NaCl (to ensure a constant humidity of the air of ca. 75%). During 15 minutes the headspace system was left equilibrating, then the volatiles were adsorbed during 15 minutes on a clean Tenax® cartridge. The sampling was repeated 7 times every hour. The cartridges were desorbed on a Perkin Elmer TurboMatrix ATD 350 desorber coupled to a Perkin Elmer Autosystem XL gas chromatograph equipped with a J&W Scientific DB1 capillary column (30 m, i.d. 0.25 mm, film 0.25 μm) and a Perkin Elmer Turbomass Upgrade mass spectrometer. The volatiles were analyzed by gas chromatography (GC) using a two steps temperature gradient starting from 70°C to 130°C at 3°C/minutes and then going to 260°C at 25°C/min. The injection temperature was at 240°C, the detector temperature at 260°C. Headspace concentrations (in ng/l air) were obtained by external standard calibrations of the corresponding fragrance aldehydes and ketones using ethanol solutions of five different concentrations. 0.1, 0.2 or 0.3 μl of the calibration solutions were injected onto Tenax® cartridges, which were immediately desorbed under the same conditions as those resulting from the headspace sampling.

The following amounts of aldehydes and ketones were detected from the sample containing the diamine derivative as compared to the reference sample without the diamine (between brackets):

| | 90 min [ng/l] | 210 min [ng/l] | 330 min [ng/l] | 450 min [ng/l] |
|---|---|---|---|---|
| Furfural | 306.4 (1.0) | 264.6 (0.3) | 203.2 (0.0) | 160.0 (0.2) |
| Citronellal | 164.2 (1.5) | 129.9 (1.1) | 76.8 (1.0) | 46.5 (1.2) |
| Trifernal® | 120.4 (4.3) | 116.8 (6.7) | 103.6 (5.9) | 87.8(5.1) |
| Delphone | 1.4 (0.7) | 0.7 (0.4) | 0.6 (0.4) | 0.5 (0.4) |
| 10-Undecenal | 152.5 (12.3) | 156.9 (23.8) | 140.2 (21.6) | 116.4 (15.2) |
| Ventral® | 69.6 (1.0) | 60.1 (1.4) | 32.7 (1.1) | 17.8 (0.9) |

The headspace concentrations of the aldehydes and ketones were found to be higher in the presence of the diamine derivative than in its absence as shown in Figure 1. The presence of the diamine has thus a positive effect on the long-lastingness of the fragrance perception on dry fabric.

<u>Example 4</u>

<u>Performance of a softener base comprising an invention's dynamic mixture</u>

An equimolar mixture (0.041 M) of the following aldehydes and ketones was obtained by weighing them into a 25 ml flask and filling up with ethanol: furfural (98.5 mg), Trifernal® (151.9 mg), Delphone (158.1 mg), 10-undecenal (172.5 mg), Vertral® (168.4 mg), 2-heptanone (117.0 mg), benzaldehyde (108.8 mg), Triplal® (141.7 mg), 4-ethylbenzaldehyde (137.5 mg), 1-(4-methylphenyl)-1-ethanone (4-methylacetophenone, 137.5 mg), decanal (151.9 mg), methoxymelonal (176.6 mg), 1,8-p-menthadien-7-al (153.8 mg), 2-methyldecanal (174.5 mg), Liminat® (170.4 mg), 3,5,5-trimethylhexanal (145.8 mg), 2,4,6-trimethyl-3-cyclohexene-1-carbaldehyde (156.0 mg) and benzylacetone (151.9 mg). The samples were prepared by adding the diamine (0.369 mmol = 18 x 0.0205 mmol) to 1.80 g of the above fabric softener base into a small vial. To another vial, serving as the reference, 1.80 g of the above fabric softener base were added. Then 0.5 ml of the solution containing equimolar amounts (0.0205 mmol) of the fragrance aldehydes and/or ketones and 1.5 ml of ethanol were added to both vials. The two samples were closed and left standing at room temperature to equilibrate. After 5 days, the samples were dispersed in a beaker with 600 ml of demineralized cold tap water, respectively. One cotton towel (cut to ca. 12 x 12 cm sheets) was added to each beaker and agitated manually for 3 minutes, left standing for 2 minutes, then wrung out by hand and weighed to obtain a constant quantity of residual water. The two towels were left drying overnight and analyzed the next day. The headspace sampling and analysis was carried out as described above (Example 2), with the exception that only 1 point was taken after 150 min (equilibration for 135 min and 15 min of adsorption on a clean Tenax® cartridge).

The following headspace concentrations were measured on dry fabric in the presence or absence (reference) of a diamine:

| | Headspace concentrations for a mixture of aldehydes and ketones [ng/l] on dry fabric after 150 min of sampling | | | | |
|---|---|---|---|---|---|
| | **A** | **B**[a] | **C** | **D**[a] | Reference[a] |
| Furfural | 1.9 | 15.8 | 91.3 | 93.3 | 2.0 |
| 2-Heptanone | 0.3 | 0.2 | 0.3 | 0.3 | 0.2 |
| Benzaldehyde | 4.8 | 230.8 | 193.5 | 250.8 | 2.2 |
| 3,5,5-Trimethylhexanal | 1.2 | 9.8 | 45.7 | 116.9 | 1.4 |
| Triplal® | 0.6 | 16.3 | 7.4 | 94.4 | 3.5 |
| 2,4,6-Trimethyl-3-cyclohexene-1-carbaldehyde | 0.6 | 4.0 | 6.4 | 6.2 | 2.5 |
| 4-Ethylbenzaldehyde | 1.0 | 68.3 | 69.1 | 103.8 | 2.4 |
| 4-Methylacetophenone | 1.1 | 1.9 | 0.5 | 2.7 | 1.9 |
| Trifernal® | 2.1 | 10.2 | 18.0 | 27.5 | 4.1 |
| Decanal | 11.4 | 20.8 | 27.6 | 47.6 | 8.3 |
| Methoxymelonal | 2.1 | 19.1 | 9.2 | 95.7 | 3.2 |
| Benzylacetone | 1.5 | 2.7 | 1.9 | 4.2 | 2.7 |
| Delphone | 0.8 | 1.4 | 0.6 | 1.9 | 1.4 |
| 1,8p-Menthadien-7-al | 1.4 | 94.4 | 58.5 | 93.6 | 2.9 |
| 2-Methyldecanal | 3.2 | 47.5 | 21.8 | 181.7 | 4.1 |
| 10-Undecenal | 5.1 | 10.8 | 16.9 | 22.9 | 6.0 |
| Liminal® | 1.6 | 11.4 | 18.1 | 48.3 | 3.3 |
| Ventral® | 2.1 | 15.1 | 17.4 | 80.2 | 3.4 |
| | Headspace concentrations for a mixture of aldehydes and ketones [ng/l] on dry fabric after 150 min of sampling | | | | |
| | **E** | **F** | **G** | **H** | Reference[a] |
| Furfural | 26.2 | 65.6 | 18.5 | 4.1 | 2.0 |
| 2-Heptanone | 0.0 | 0.0 | 0.3 | 0.0 | 0.2 |
| Benzaldehyde | 168.7 | 329.2 | 180.8 | 28.6 | 2.2 |
| 3,5,5-Trimethylhexanal | 101.3 | 110.9 | 17.9 | 3.6 | 1.4 |
| Triplal® | 156.1 | 173.9 | 23.6 | 5.8 | 3.5 |
| 2,4,6-Trimethyl-3-cyclohexene-1-carbaldehyde | 7.1 | 6.7 | 8.1 | 6.6 | 2.5 |

(continued)

| | Headspace concentrations for a mixture of aldehydes and ketones [ng/l] on dry fabric after 150 min of sampling | | | | |
|---|---|---|---|---|---|
| | E | F | G | H | Reference[a] |
| 4-Ethylbenzaldehyde | 6.8 | 94.0 | 64.8 | 35.1 | 2.4 |
| 4-Methylacetophenone | 4.8 | 4.8 | 1.2 | 4.4 | 1.9 |
| Triferal® | 16.0 | 13.0 | 14.4 | 6.2 | 4.1 |
| Decanal | 16.2 | 20.6 | 35.0 | 15.1 | 8.3 |
| Methoxymelonal | 21.5 | 33.0 | 39.3 | 6.0 | 3.2 |
| Benzylacetone | 6.5 | 6.0 | 2.1 | 4.8 | 2.7 |
| Delphone | 3.1 | 3.4 | 0.9 | 3.1 | 1.4 |
| 1,8-p-Menthadien-7-al | 6.6 | 107.7 | 48.9 | 38.5 | 2.9 |
| 2-Methyldecanal | 17.3 | 100.1 | 156.3 | 24.4 | 4.1 |
| 10-Undecenal | 7.4 | 10.1 | 20.1 | 7.8 | 6.0 |
| Liminal® | 20.0 | 17.5 | 25.8 | 7.3 | 3.3 |
| Vertral® | 93.9 | 72.7 | 45.6 | 6.9 | 3.4 |

[a] average values of at least two measurements.
**A** = N,N'-dimethylethane-1,2-diamine; **B** = N,N'-dibenzylethane-1,2-diamine; **C** = N,N'-dibenzylpropane-1,3-diamine; **D =** (1*R*,2*R*)-N,N'-dibenzylcyclohexane-1,2-diamine.
[a] average values of at least two measurements.
**E** = (1*R*,2*S*)-N,N'-dibenzylcyclohexane-1,2-diamine; **F** = (cis/trans)-N,N'-dibenzylcyclohexane-1,2-diamine; **G** = N-benzyl-N-(2-piperidinylmethyl)amine; **H** = N,N'-bis(4-methoxybenzyle)ethane-1,2-diamine.

| | Headspace concentrations for a mixture of aldehydes and ketones [ng/l] on dry fabric after 150 min of sampling | | | | |
|---|---|---|---|---|---|
| | I | J | K | L | Reference[a] |
| Furfural | 55.0 | 42.4 | 58.8 | 31.8 | 2.0 |
| 2-Heptanone | 0.0 | 0.3 | 0.2 | 0.0 | 0.2 |
| Benzaldehyde | 81.9 | 127.1 | 67.0 | 50.1 | 2.2 |
| 3,5,5-Trimethylhexanal | 28.0 | 30.8 | 22.7 | 8.0 | 1.4 |
| Triplal® | 5.8 | 16.8 | 0.8 | 11.5 | 3.5 |
| 2,4,6-Trimethyl-3-cyclohexene-1-carbaldehyde | 6.6 | 4.2 | 10.3 | 6.6 | 2.5 |
| 4-Ethylbenzaldehyde | 119.1 | 83.5 | 53.4 | 75.5 | 2.4 |
| 4-Methylacetophenone | 4.6 | 1.7 | 0.8 | 4.5 | 1.9 |
| Triferal® | 23.9 | 25.7 | 26.9 | 6.6 | 4.1 |
| Decanal | 37.5 | 33.3 | 24.4 | 12.0 | 8.3 |
| Methoxymelonal | 5.0 | 68.1 | 3.1 | 20.8 | 3.2 |
| Benzylacetone | 5.2 | 4.4 | 2.0 | 4.8 | 2.7 |
| Delphone | 3.1 | 0.9 | 0.7 | 3.1 | 1.4 |
| 1,8-p-Menthadien-7-al | 111.6 | 76.0 | 18.2 | 35.9 | 2.9 |
| 2-Methyldecanal | 4.1 | 146.6 | 3.9 | 25.4 | 4.1 |

(continued)

| | Headspace concentrations for a mixture of aldehydes and ketones [ng/l] on dry fabric after 150 min of sampling | | | | |
|---|---|---|---|---|---|
| | **I** | **J** | **K** | **L** | Reference[a] |
| 10-Undecenal | 33.2 | 24.0 | 23.2 | 6.7 | 6.0 |
| Liminal® | 24.8 | 32.8 | 19.0 | 7.5 | 3.3 |
| Vertral® | 7.0 | 65.1 | 3.4 | 8.6 | 3.4 |

[a]average values of at least two measurements.
**I** = N,N'-bis(4-methoxybenzyl)propane-1,3-diamine; **J** = N,N'-bis[4-(dimethylamino)benzyl]-ethane-1,2-diamine; **K** = N,N'-bis[4-(dimethylamino)benzyl]propane-1,3-diamine; **L =** N,N'-bis(4-ethylbenzyl)ethane-1,2-diamine.

| | Headspace concentrations for a mixture of aldehydes and ketones [ng/l] on dry fabric after 150 min of sampling | | | | |
|---|---|---|---|---|---|
| | **M** | **N** | **O**[b] | **P**[b] | Reference[a] |
| Furfural | 8.3 | 4.1 | 4.3 | 3.2 | 2.0 |
| 2-Heptanone | 0.0 | 0.0 | 0.0 | 0.2 | 0.2 |
| Benzaldehyde | 136.0 | 4.8 | 84.1 | 48.2 | 2.2 |
| 3,5,5-Trimethylhexanal | 41.3 | 12.7 | 8.6 | 5.4 | 1.4 |
| Triplal® | 66.3 | 5.8 | 5.7 | 1.0 | 3.5 |
| 2,4,6-Trimethyl-3-cyclohexene-1-carbaldehyde | 6.8 | 6.6 | 6.6 | 0.5 | 2.5 |
| 4-Ethylbenzaldehyde | 84.1 | 5.5 | 6.4 | 12.6 | 2.4 |
| 4-Methylacetophenone | 4.5 | 4.4 | 4.5 | 0.6 | 1.9 |
| Trifernal® | 15.5 | 11.2 | 12.3 | 17.0 | 4.1 |
| Decanal | 38.0 | 35.3 | 17.7 | 28.8 | 8.3 |
| Methoxymelonal | 33.8 | 10.5 | 8.5 | 10.0 | 3.2 |
| Benzylacetone | 4.9 | 4.9 | 5.0 | 1.8 | 2.7 |
| Delphone | 3.1 | 3.1 | 3.1 | 0.6 | 1.4 |
| 1,8-p-Menthadien-7-al | 105.2 | 6.5 | 7.2 | 2.7 | 2.9 |
| 2-Methyldecanal | 96.6 | 15.8 | 11.9 | 15.5 | 4.1 |
| 10-Undecenal | 19.0 | 26.5 | 14.5 | 47.9 | 6.0 |
| Liminal® | 27.6 | 9.8 | 9.1 | 14.6 | 3.3 |
| Ventral® | 42.9 | 8.9 | 7.1 | 3.3 | 3.4 |

[a] average values of at least two measurements;[b] used at 0.25 molar equivalents with respect to the molar amount of the sum of aldehydes and ketones.
**M** = dimethyl 4,4'-[1,2-ethanedlylbis(iminomethylene)]dibenzoate; **N** = piperazine; **O** = (1*RS*,2*SR*)-N,N'-dibenzyl-1,2-diphenylethane-1,2-diamine; **P** = (1*R*,2*R*)-N,N'-dibenzylcyclohexane-1,2-diamine.

The data show that the presence of a diamine increases the headspace concentrations of the volatile aldehydes (and to a lower extent of the ketones) from the mixture. The nature of the substituent at the N-atom of the diamine is very important for the performance of the dynamic mixture. Whereas the presence of N,N'-dimethylethane-1,2-diamine **(A)** in the mixture had almost no influence on the headspace concentration of the volatiles (the headspace concentrations remained below 15 ng/l), considerably higher headspace concentrations (in some cases above 150 ng/l) were obtained

with benzyl **(B-G)** or substituted benzyl **(H-M)** residues at the N-atom of the diamine (Figure 3). Similarly, cyclic diamines **(C-G)** give rise to higher headspace concentrations than acyclic diamines.

For example, the headspace concentration of benzaldehyde increased by a factor of 2 in the presence of diamine **A,** by a factor of ca. 90 with diamines **B** or **C,** by a factor of 114 with diamine **D,** by a factor of 77 with diamine **E,** by a factor of 150 with diamine **F,** by a factor of 82 with diamine **G,** by a factor of 13 with diamine **H,** by a factor of 37 with diamine **I,** by a factor of 58 with diamine **J** and by a factor of 30 with diamine **K,** by a factor of 23 with diamine **L,** by a factor of 62 with diamine **M** and by a factor of 50 (data not shown) with (cis/trans)-N,N'-dibenzylcyclohexane-1,3-diamine as compared to the reference sample. The presence of at least one benzyl or substituted benzyl group at the N-atom of the diamine thus increases the headspace concentration of the volatile carbonyl compounds by one or two orders of magnitude.

Reducing the amount of diamine with respect to the fragrance aldehydes and ketones still gave higher headspace concentrations as compared to the reference, as it was seen for the example of (1*RS*,2*SR*)-N,N'-dibenzyl-1,2-diphenylethane-1,2-diamine **(O)** and (1*R*,2*R*)-N,N'-dibenzylcyclohexane-1,2-diamine **(P),** when only half, or even only a quarter of the above mentioned amount of diamine was added to the fabric softener. In the presence of only one quarter of the molar equivalent of diamines **O** and **P** the headspace concentration of benzaldehyde increased by a factor of 38 or 22, respectively.

Example 5

Washing cycle using a softener base comprising an invention's dynamic mixture:

The use as perfuming ingredient of the present invention's dynamic mixtures in a softener base was tested by olfactive evaluation on fabric after a machine-washing cycle.

A fabric softener base with the following final composition has been prepared:

| | |
|---|---|
| Stepantex® VL 90A (origin: Stepan) | 16.5 % by weight |
| Calcium chloride (10% in demineralized water) | 0.6 % by weight |
| Demineralized water | 82.9 % by weight |

Dynamic mixtures were prepared by adding 156.0 mg of 2-methylundecanal and 232.2 mg of (cis/trans)-N,N'-dibenzylcyclohexane-1,2-diamine to 34.6 g (= 35 ml) of the fabric softener base. A second sample containing 156.2 mg of of 2-methylundecanal in 34.6 g of the softener base was prepared as a reference sample without diamine. Similarly, a second pair of samples was prepared, using 109.2 mg of Triplal® and 231.8 mg of (cis/trans)-N,N'-dibenzylcyclohexane-1,2-diamine in 34.6 g of softener base and 109.2 mg of Triplal® in 34.6 g of softener base as the reference without diamine. The resulting samples were shaken, and then left equilibrating for 5 d at room temperature prior to a use in a wash test.

30 small cotton terry towels (28 x 28 cm, in total ca. 1.4 kg) were washed together with other cotton or synthetic fabric (ca. 1.0 kg) in a Miele W26-23 washing machine. A total of 85 g of an unperfumed detergent powder (Via, origin: Unilever, Stockholm, Sweden, placed in a small container) was added to the fabric. The towels were washed in a short cycle at 40°C with 600 RPM (rotations per minute) for the spinning cycle. As soon as the machine was drawing water, a solution of 35 g of the fabric softener bases (containing either one of the above mentioned dynamic mixtures or the corresponding reference) diluted with water was added via the dispensing tray. Once the cycle was finished the cotton terry towels were line dried (at 22°C and 60% humidity) for 24 h and then evaluated by 30 panelists.

The samples were evaluated in pairs in a blind test (using one sample containing the diamine and the other the coresponding reference) by ranking the odor intensity of the two samples on a linear scale between 0 ("odorless") and 10 ("very strong odor"). The following results were obtained:

| Sample | 2-methylundecanal + diamine | 2-methylundecanal (reference) | Triplal® + diamine | Trivial® (reference) |
|---|---|---|---|---|
| Average intensity (0-10) | 6.45 | 2.64 | 6.39 | 2.92 |
| Standard deviation (n-1) | 2.29 | 1.65 | 2.57 | 2.60 |
| Confidence interval (at 95%) | 0.85 | 0.62 | 0.96 | 0.97 |

The panelists detected a strong difference between the two samples of each pair, with the sample containing the diamine being significantly stronger than the reference, thus confirming the desired controlled release effect of the

active aldehyde.

Example 6

Performance of a shampoo base comprising an invention's dynamic mixture:

The use as perfuming ingredient of the present invention's mixtures has been tested in a shampoo application on hair swatches.

A shampoo base with the following final composition has been prepared:

| | |
|---|---|
| Texapon® NSO IS, sodium laureth sulfate (origin: Henkel) | 48.0 % by weight |
| Dehyton® AB-30, coco-betaine (origin: Henkel) | 7.0 % by weight |
| Dow Coming 2-1691 Emulsion (origin: Dow Coming) | 3.0 % by weight |
| Rewomid IPP 240, cocamide MIPA (origin: Witco Surfactants) | 1.2 % by weight |
| Cetyl alcohol | 1.2 % by weight |
| Cithrol EGDS 3432, ethylene glycol distearate (origin: Croda) | 0.7 % by weight |
| Jaguar Excel, guar hydroxypropyltrimmonium chloride (origin: Rhodia) | 0.4 % by weight |
| Glydant® Plus Liquid, preservative (origin: Lonza) | 0.3 % by weight |
| Deionized water | 38.2 % by weight |

The perfuming performance, over time, of the free perfuming aldehydes/ketones and of the invention's mixtures (i.e. the free perfuming aldehydes/ketones with a diamine derivative as additive) has been determined in the following experiment:

The above shampoo base (2.00 g) was weighed into two small vials, respectively. Then 200 μl of a solution containing equimolar amounts (0.6 mmol) of 2-methylundecanal (110.3 mg), Triplal® (83.0 mg), 3,5,5-trimethylhexanal (86.8 mg), benzaldehyde (63.9 mg), methoxymelonal (104.2 mg) and Vertral® (98.2 mg) in 10 ml of ethanol were added to each vial. Furthermore, to one of the samples 21.17 mg (0.072 mmol) of (cis/trans)-N,N'-dibenzylcyclohexane-1,2-diamine were added. The two samples were then closed and left standing at room temperature to equilibrate for 5 d. Two hair swatches (ca. 5 g, origin: A. & C. Sécher Fesnoux, Industrie du cheveu, Chaville, France) were wetted with tap water (at ca. 35°C), washed with 1.0 g of the above mentioned unperfumed shampoo base and rinsed with water, respectively. One of the hair swatches were then washed for 1 min with 0.5 g of the shampoo base containing the perfumery aldehydes together with the diamine derivative, the other with 0.5 g of the shampoo base containing only the perfumery aldehydes. The hair swatches were each rinsed for 30 s. The washing was repeated a second time with another 0.5 g of the respective shampoo bases. After leaving for 2 min, the swatches were rinsed with water (at 25°C) for 1 min and pre-dried shortly with household paper. The swatches were left drying overnight and analyzed the next day. Each hair swatch was put into a headspace sampling cell (160 ml) thermostatted at 25°C and exposed to a constant air flow of 200 ml/min, respectively. The air was filtered through active charcoal and aspirated through a saturated solution of NaCl (to ensure a constant humidity of the air of ca. 75%). During 55 min the headspace system was left equilibrating, then the volatiles were adsorbed during 15 min (dry swatches) on a clean Tenax® cartridge. The sampling was repeated 7 times every 30 min. The cartridges were desorbed on a Perkin Elmer TurboMatrix ATD 350 desorber coupled to a Perkin Elmer Autosystem XL gas chromatograph equipped with a J&W Scientific DB1 capillary column (30 m, i.d. 0.25 mm, film 0.25 μm) and a Perkin Elmer Turbomass Upgrade mass spectrometer. The volatiles were analyzed by GC using a two-step temperature gradient starting from 70°C to 130°C at 3°C/minutes and then going to 260°C at 25°C/min. The injection temperature was at 240°C, the detector temperature at 260°C. Headspace concentrations (in ng/l) were obtained by external standard calibrations of the corresponding fragrance aldehydes using ethanol solutions of five different concentrations. 2 μl of each calibration solution was injected onto Tenax® cartridges, which were immediately desorbed under the same conditions as those resulting from the headspace sampling.

The following amounts of aldehydes were detected in the headspace of the sample containing (cis/trans)-N, N'-dibenzylcyclohexane-1,2-diamine as compared to the reference sample without the diamine (between brackets):

| | 90 min [ng/l] | 210 min [ng/l] | 330 min [ng/l] | 450 min [ng/l] |
|---|---|---|---|---|
| 2-Methylundecanal | 10.0 (4.7) | 15.1 (6.2) | 15.9 (7.4) | 13.9 (7.9) |
| Triplal® | 10.1 (7.5) | 10.1 (9.1) | 9.3 (8.4) | 10.6 (8.6) |
| 3,5,5-Trimethylhexanal | 8.9 (5.0) | 10.9 (2.3) | 11.0 (2.6) | 11.1 (2.4) |
| Benzaldehyde | 32.9 (3.2) | 42.6 (3.1) | 40.1 (3.1) | 34.6 (3.6) |
| Methoxymelonal | 6.5 (4.9) | 7.6 (4.4) | 6.1 (4.5) | 6.3 (4.5) |
| Vertral® | 11.9 (9.8) | 9.7 (10.1) | 13.0 (9.9) | 11.6 (10.3) |

Slightly higher or at least comparable headspace concentrations were measured for the sample containing the diamine as compared to the reference sample. In the case of benzaldehyde, 3,5,5-trimethylhexanal or 2-methylundecanal the presence of the diamine increased the headspace concentrations by a factor of *ca.* 10, 3 and 2, respectively. The data illustrate that the presence of the diamine derivative has a positive effect on the long-lastingness of the fragrance aldehydes in a typical shampoo application.

Example 7

Performance of an air freshener gel comprising an invention's dynamic mixture:

The use as perfuming ingredient of the present invention's mixtures has been tested in an air freshener gel.
A gel with the following final composition has been prepared:

| | |
|---|---|
| Satiagel® | 1.5 % by weight |
| Nipasol® M Sodium (sodium propylparaben) | 0.5 % by weight |
| Deionized water | 96.8 % by weight |

The perfuming performance, over time, of the free perfuming aldehydes/ketones and of the invention's mixtures (i.e. the free perfuming aldehydes/ketones with a diamine derivative as additive) has been determined in the following experiment:
The above gel base (4.94 g) was weighed into two 10 ml glass vials, respectively. The gel was melted by heating the vials to 80°C on a water bath. Then 0.01 g (= 0.2 % by weight) of a surfactant (Tween® 20, origin: Fluka) and 0.05 g (= 1.0 % by weight) of a mixture of aldehydes containing equimolar amounts (2.0 mmol) of 3,5,5-trimethylhexanal (284.0 mg), Triplal® (276.0 mg), methoxymelonal (345.0 mg), Ventral® (328.5 mg), 4-ethylbenzaldehyde (267.8 mg) and 2-methylundecanal (368.4 mg) were added to each vial. Furthermore, to one of the samples 94.1 mg (0.32 mmol) of (cis/trans)-N,N'-dibenzylcyclohexane-1,2-diamine were added. The two samples were then left cooling to room temperature (formation of the gel) and left standing at the air for several weeks. At different time intervals, the vials were put into an headspace sampling cell (625 ml) and exposed to a constant air flow of ca. 200 ml/min, respectively. The air was filtered through active charcoal and aspirated through a saturated solution of NaCl (to ensure a constant humidity of the air of ca. 75%). During 30 min the headspace system was left equilibrating, then the volatiles were adsorbed during 20 min (after 4 and 18 d) or 30 min (after 53 d) on a clean Tenax® cartridge, respectively. The cartridges were desorbed on a Perkin Elmer TurboMatrix ATD desorber coupled to a Carlo Erba MFC 500 gas chromatograph equipped with a J&W Scientific DB1 capillary column (30 m, i.d. 0.45 mm, film 0.42 μm) and a FID detector. The volatiles were analyzed by GC using a two step temperature gradient starting from 70°C to 130°C at 3°C/min and then going to 260°C at 25°C/min. The injection temperature was at 240°C, the detector temperature at 260°C.
The following amounts of aldehydes (rel. GC peak areas) were detected in the headspace of the sample containing (cis/trans)-N,N'-dibenzylcyclohexane-1,2-diamine as compared to the reference sample without the diamine (between brackets):

| | 4 d x $10^3$ [peak area] | 18d x $10^3$ [peak area] | 53 d x $10^3$ [peak area] |
|---|---|---|---|
| 3,5,5-Trimethylhexanal | 1005 (4704) | 687 (1677) | 350 (138) |
| Triplal® | 237(2221) | 177(112) | 67(23) |

(continued)

| | 4 d x $10^3$ [peak area] | 18d x $10^3$ [peak area] | 53 d x $10^3$ [peak area] |
|---|---|---|---|
| 4-Ethylbenzaldehyde | 399 (6657) | 341 (3935) | 91 (123) |
| Methoxymelonal | 141 (893) | 169 (140) | 92 (15) |
| Vertral® | 52 (179) | 289 (341) | 217 (107) |
| 2-Methylundecanal | 0 (80) | 0 (49) | 0 (0) |

The data show that the presence of the diamine according to the invention influences the evaporation profile of the different aldehydes from the gel. In most of the cases, the decrease of the fragrance evaporation is less pronounced (or more steady) in the presence of the diamine, as compared to the reference sample without diamine. With the exception of 4-ethylbenzaldehyde, this results in higher amounts of aldehydes in the headspace of the sample containing the diamine after 53 d and thus illustrates a slow fragrance release effect in the presence of the diamine over time. The diamines according to the present invention are therefore suitable compounds for the use in air freshener applications.

## Claims

**1.** Use as perfuming ingredient of a dynamic mixture, for the controlled release of active aldehydes or ketones, obtainable by reacting, in a water-containing medium,

i) at least one active aldehyde or ketone having a molecular weight comprised between 80 and 230 g/mol and being a perfuming, flavoring, insect repellent or attractant ingredient, being selected from the group consisting of the $C_{5-20}$ perfuming aldehydes and the $C_{5-20}$ perfuming ketones;
with
ii) at least one derivative of formula

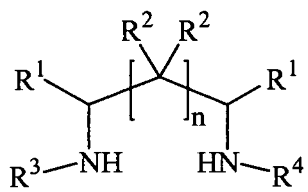

(I)

wherein:

n represents an integer varying from 0 to 3;
$R^1$ represent, independently of each other, a hydrogen atom, a phenyl group optionally substituted, or a $C_{1-18}$ alkyl or alkenyl group optionally substituted;
$R^2$ represent, independently of each other, a hydrogen atom, a phenyl group optionally substituted, or a $C_{1-6}$ alkyl or alkenyl group optionally substituted; two $R^2$ or two $R^1$ or one $R^1$ and one $R^2$, taken together, may form a $C_{3-5}$ alkanediyl or alkenediyl group; and
$R^3$ and $R^4$ represent each a $C_{1-3}$ alkyl group substituted by a phenyl group optionally substituted; $R^3$ and $R^4$ or $R^3$ and the adjacent $R^1$, taken together, may form a $C_{2-4}$ alkanediyl or alkenediyl group.

**2.** Use according to claim 1, **characterized in that** the derivative of formula (I) is a compound wherein:

- the $R^3$ group is taken together with the adjacent $R^1$ to form an alkanediyl or alkenediyl group as defined in claim 1; or
- two $R^1$ groups, or two $R^2$ groups or one $R^2$ and one $R^1$ group, are taken together to form a group as defined in claim 1.

**3.** Use according to claim 1, **characterized in that** the derivative of formula (I)
is a compound of formula

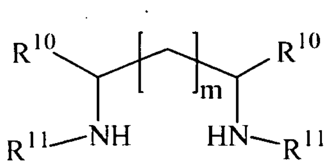

(II)

wherein m represents 0 or 1;
$R^{10}$ represent, independently of each other, a hydrogen atom, a phenyl group optionally substituted, or a $C_{1-4}$ alkyl group optionally substituted; the two $R^{10}$, taken together, may form a $C_{3-4}$ alkanediyl or alkenediyl group; and
$R^{11}$ represent, independently of each other, a $C_{1-3}$ alkyl group substituted by a phenyl group optionally substituted; two $R^{11}$ groups or one $R^{10}$ and one $R^{11}$ group, taken together, may form a $C_{2-4}$ alkanediyl or alkenediyl group.

**4.** Use according to claim 1, **characterized in that** the derivative of formula (I) is selected amongst:

i) $BzNHCH_2(CH_2)_gCH_2NHBz$, wherein g is 1 or 0 and Bz is a substituted or non-substituted benzyl group;
ii) $R^{12}HN\text{-}(C_6H_{10})NHR^{12}$ wherein $R^{12}$ is a Bz group as defined above;
iii) piperazine or 1,4-diaza-cycloheptane;
iv) $R^{12}HNCHArCHArNHR^{12}$, wherein $R^{12}$ is a Bz group as defined above and Ar is a phenyl group; or
v) $(C_5H_9NH)CH_2NHR^{12}$ wherein $R^{12}$ is a Bz group as defined above.

**5.** Use according to claim 1, **characterized in that** the derivative of formula (I) is selected amongst:

N,N'-dibenzylethane-1,2-diamine (N,N'-dibenzylethylenediamine), N,N'-dibenzylpropane-1,3-diamine, N,N'-dibenzylcyclohexane-1,2-diamine, N,N'-bis[4-(dimethylamino)benzyl]ethane-1,2-diamine, N,N'-bis[4-(dimethylamino)benzyl]propane-1,3-diamine, N,N'-bis(4-methoxybenzyl)ethane-1,2-diamine, N,N'-bis(4-methoxybenzyl)propane-1,3-diamine, dimethyl or diethyl 4,4'-[1,2-ethanediylbis(iminomethylene)]dibenzoate, N,N'-bis(4-ethylbenzyl)ethane-1,2-diamine, N,N'-dibenzyl- 1,2-diphenylethane- ,2-diamine or N-benzyl-N-(2-piperidinylmethyl)amine.

**6.** Use according to any one of claims 1 to 5, **characterized in that** the active aldehyde or ketone is a perfuming aldehyde or ketone.

**7.** Use according to claim 6, **characterized in that** the perfuming aldehyde or ketone is **characterized by** a vapor pressure above 2.0 Pa.

**8.** A dynamic mixture obtainable by reacting, in a water-containing medium,

i) at least two active aldehyde or ketone having a molecular weight comprised between 80 and 230 g/mol and being a perfuming, flavoring, insect repellent or attractant ingredient, being selected from the group consisting of the $C_{5-20}$ perfuming aldehydes and the $C_{5-20}$ perfuming ketones;
with
ii) at least one derivative of formula

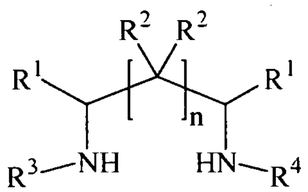

(I)

wherein:

n represents an integer varying from 0 to 3;
$R^1$ represent, independently of each other, a hydrogen atom, a phenyl group optionally substituted, or a $C_{1-18}$

alkyl or alkenyl group optionally substituted; $R^2$ represent, independently of each other a hydrogen atom, a phenyl group optionally substituted, or a $C_{1-6}$ alkyl or alkenyl group optionally substituted; two $R^2$ or two $R^1$ or one $R^1$ and one $R^2$, taken together, may form a $C_{3-5}$ alkanediyl or alkenediyl group; and $R^3$ and $R^4$ represent each a $C_{1-3}$ alkyl group substituted by a phenyl group optionally substituted; $R^3$ and $R^4$ or $R^3$ and the adjacent $R^1$, taken together, may form a $C_{2-4}$ alkanediyl or alkenediyl group.

**9.** An aminal of formula

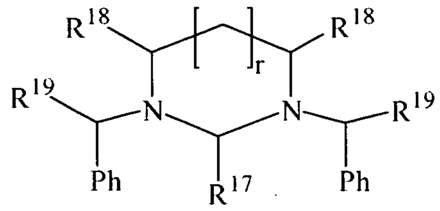

(IV)

wherein r represents 0 or 1; $R^{19}$ represent, independently of each other, a hydrogen atom or a methyl or ethyl group; $R^{18}$ represent, independently for each other, a hydrogen atom, a phenyl group optionally substituted by one or two OH or $C_1$-$C_4$ alkyl or alkoxyl groups, or a $C_{1-4}$ alkyl group; two $R^{18}$, taken together, may form a $C_{3-4}$ alkanediyl or alkenediyl group; Ph represent, independently for each other, a phenyl group optionally substituted by one or two $NR^{20}_2$, $(NR^{20}_3)X$, $OR^{20}$, $SO_3M$, $COOR^{20}$ or $R^{20}$, with $R^{20}$ representing a $C_1$ to $C_3$ or $C_4$ alkyl group or a hydrogen atom, M representing a hydrogen atom or an alkali metal ion, and X representing a halogen atom or a sulphate; and $R^{17}$ is the residue of an active aldehyde $R^{17}CHO$ having a molecular weight comprised between 80 and 230 g/mol and being a perfuming, flavoring, insect repellent or attractant ingredient, and wherein $R^{17}$ represents a $C_6$-$C_{14}$ alkyl, alkenyl or alkadienyl group optionally substituted by an OH or an $OR^{15}$ group, or a $C_{1-3}$ alkyl or alkenyl group substituted by a phenyl group optionally substituted by one, two or three OH, $R^{15}$ or $OR^{15}$ groups, $R^{15}$ being an acetyl or a $C_1$-$C_4$ alkyl or alkenyl group; provided that if Ph is substituted with OH or OMe groups and $R^{18}$ and $R^{19}$ are hydrogen atoms, then said $R^{17}$ represents

- a $C_7$-$C_{14}$ alkyl group or a $C_6$-$C_{14}$ alkenyl, alkadienyl group,
- a $C_{1-3}$ alkyl group substituted by a phenyl group substituted by one, two or three OH, $R^{15}$ or $OR^{15}$ groups,
- $C_{2-3}$ alkyl group substituted by a phenyl group or
- a $C_{2-3}$ alkenyl group substituted by a phenyl group substituted by one, two or three OH, $R^{15}$ or $OR^{15}$ groups,

$R^{15}$ being a $C_1$-$C_4$ alkyl or alkenyl group; and provided that 1,2,3-tribenzyl-imidazolidine, 1,3-dibenzyl-2-styryl-imidazolidine, 1,3-dibenzyl-2-hexyl-imidazolidine and 1,3-bis(4-dimethylaminobenzyl)-2-styryl-imidazolidine are excluded.

**10.** A perfuming composition comprising:

i) as perfuming ingredient, a dynamic mixture as defined in claim 1;
ii) at least one ingredient selected from the group consisting of a perfumery carrier and a perfumery base; and
iii) optionally at least one perfumery adjuvant.

**11.** A perfumed article comprising:

i) as perfuming ingredient, a dynamic mixture as defined in claim 1; and
ii) a liquid consumer product base.

**12.** A perfumed article according to claim 11, **characterized in that** the liquid consumer product base is a perfume, cologne or after-shave lotion, a perfumed soap, a detergent, a shower or bath mousse, oil or gel, a hygiene product or hair care product, a body-care product, a deodorant or antiperspirant, an air freshener, a cosmetic preparation, a fabric refresher, an ironing water, a paper, a wipe or bleach, a softener base.

**13.** A perfumed article according to claim 12, **characterized in that** the derivative of formula (1) is N,N'-dibenzylcyclohexane-1,2-diamine or N-benzyl-N-(2-piperidinylmethyl)amine.

**14.** A perfumed article comprising:

i)

- a derivative of formula (I), as defined in claim 1, and/or at least one aminal obtainable from a derivative of formula (I) and an active aldehyde or ketone, as defined in claim 1, and
a perfume or perfuming composition containing at least one perfuming aldehyde or ketone having a molecular weight comprised between 80 and 230 g/mol; or
- at least one aminal obtainable from a derivative of formula (I) and an active aldehyde

or ketone;
and
ii) a solid consumer product base intended to be used in the presence of water.

**15.** A perfumed article according to claim 14, **characterized in that** the solid consumer product base is a perfumed soap, a detergent, a hygiene product, a body-care product, a deodorant or antiperspirant, an air freshener, a cosmetic preparation,, a paper, a wipe or a bleach.

**16.** A perfumed article according to claim 15, **characterized in that** the derivative of formula (I) is N,N'-dibenzylcyclohexane-1,2-diamine or N-benzyl-N-(2-piperidinylmethyl)amine.

## Patentansprüche

**1.** Verwendung als Duftstoffbestandteil eines dynamischen Gemischs für die kontrollierte Freisetzung aktiver Aldehyde oder Ketone, das durch zur Reaktion bringen in einem Wasser enthaltenden Medium von

i) mindestens einem aktiven Aldehyd oder Keton, der/das ein Molekulargewicht zwischen 80 und 230 g/mol aufweist und ein Duftstoff-, Geschmacksstoff-, Insektenabwehrmittel- oder -lockmittelbestandteil ist, ausgewählt aus der Gruppe, die aus den $C_{5-20}$-Duftstoffaldehyden und den $C_{5-20}$-Duftstoffketonen besteht;
mit
ii) mindestens einem Derivat der Formel

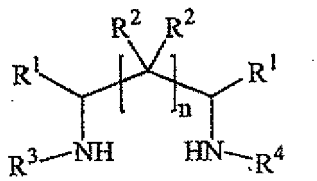

(I)

erhältlich ist, worin:

n eine von 0 bis 3 variierende ganze Zahl darstellt;
$R^1$, unabhängig voneinander, ein Wasserstoffatom, eine optional substituierte Phenylgruppe oder eine optional substituierte $C_{1-18}$-Alkyl- oder -Alkenylgruppe darstellen;
$R^2$, unabhängig voneinander, ein Wasserstoffatom, eine optional substituierte Phenylgruppe oder eine optional substituierte $C_{1-6}$-Alkyl- oder -Alkenylgruppe darstellen; zwei $R^2$ oder zwei $R^1$ oder ein $R^1$ und ein $R^2$, zusammen genommen, eine $C_{3-5}$-Alkandiyl- oder -Alkendiylgruppe bilden können;
und
$R^3$ und $R^4$ jeweils eine $C_{1-3}$-Alkylgruppe, substituiert durch eine optional substituierte Phenylgruppe darstellen; $R^3$ und $R^4$ oder $R^3$ und das benachbarte $R^1$, zusammen genommen, eine $C_{2-4}$-Alkandiyl- oder -Alkendiylgruppe bilden können.

**2.** Verwendung nach Anspruch 1, **dadurch gekennzeichnet, dass** das Derivat der Formel (I) eine Verbindung ist,

worin:

- die $R^3$-Gruppe mit dem benachbarten $R^1$ zur Bildung einer wie nach Anspruch 1 definierten Alkandiyl- oder -Alkendiylgruppe zusammen genommen ist; oder
- zwei $R^1$-Gruppen, oder zwei $R^2$-Gruppen oder eine $R^2$- und eine $R^1$-Gruppe zur Bildung einer wie nach Anspruch 1 definierten Gruppe zusammen genommen sind.

**3.** Verwendung nach Anspruch 1, **dadurch gekennzeichnet, dass** das Derivat der Formel (I) eine Verbindung der Formel

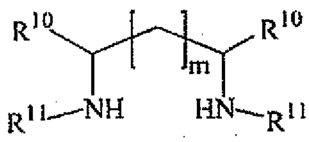

(II)

ist, worin m 0 oder 1 darstellt;
$R^{10}$, unabhängig voneinander, ein Wasserstoffatom, eine optional substituierte Phenylgruppe oder eine optional substituierte $C_{1-4}$-Alkylgruppe darstellen; die beiden $R^{10}$, zusammen genommen, eine $C_{3-4}$-Alkandiyl- oder -Alkendiylgruppe bilden können; und
$R^{11}$, unabhängig voneinander, eine $C_{1-3}$-Alkylgruppe, substituiert durch eine optional substituierte Phenylgruppe darstellen; zwei $R^{11}$-Gruppen oder eine $R^{10}$- und eine $R^{11}$-Gruppe, zusammen genommen, eine $C_{2-4}$-Alkandiyl- oder -Alkendiylgruppe bilden können.

**4.** Verwendung nach Anspruch 1, **dadurch gekennzeichnet, dass** das Derivat der Formel (I) ausgewählt ist unter:

i) $BzNHCH_2(CH_2)_gCH_2NHBz$, worin g 1 oder 0 ist und Bz eine substituierte oder nicht substituierte Benzylgruppe ist;
ii) $R^{12}HN-(C_6H_{10})NHR^{12}$, worin $R^{12}$ eine wie vorstehend definierte Bz-Gruppe ist;
iii) Piperazin oder 1,4-Diaza-cycloheptan;
iv) $R^{12}HNCHArCHArNHR^{12}$, worin $R^{12}$ eine wie vorstehend definierte Bz-Gruppe ist und Ar eine Phenylgruppe ist; oder
v) $(C_5H_9NH)CH_2NHR^{12}$, worin $R^{12}$ eine wie vorstehend definierte Bz-Gruppe ist.

**5.** Verwendung nach Anspruch 1, **dadurch gekennzeichnet, dass** das Derivat der Formel (I) ausgewählt ist unter:

N,N'-Dibenzylethan-1,2-diamin (N,N'-Dibenzylethylendiamin), N,N'-Dibenzylpropan-1,3-diamin, N,N'-Dibenzylcyclohexan-1,2-diamin, N,N'-Bis[4-(dimethylamino)benzyl]ethan-1,2-diamin, N,N'-Bis[4-(dimethylamino)benzyl]propan-1,3-diamin, N,N'-Bis(4-methoxybenzyl)ethan-1,2-diamin, N,N'-Bis(4-methoxybenzyl)propan-1,3-diamin, Dimethyl- oder Diethyl-4,4'-[1,2-ethandiyl-bis(iminomethylen)]dibenzoat, N,N'-Bis(4-ethylbenzyl)ethan-1,2-diamin, N,N'-Dibenzyl-1,2-diphenylethan-1,2-diamin oder N-Benzyl-N-(2-piperidinylmethyl)amin.

**6.** Verwendung nach einem der Ansprüche 1 bis 5, **dadurch gekennzeichnet, dass** der aktive Aldehyd oder das aktive Keton ein Duftstoffaldehyd oder -keton ist.

**7.** Verwendung nach Anspruch 6, **dadurch gekennzeichnet, dass** der Duftstoffaldehyd oder das Duftstoffketon durch einen Dampfdruck von über 2,0Pa **gekennzeichnet** ist.

**8.** Dynamisches Gemisch, das durch zur Reaktion bringen in einem Wasser enthaltenden Medium

i) von mindestens zwei aktiven Aldehyden oder Ketonen, die ein Molekulargewicht zwischen 80 und 230 g/mol aufweisen und ein Duftstoff-, Geschmacksstoff , Insektenabwehrmittel- oder -lockmittelbestandteil sind, ausgewählt aus der Gruppe, die aus den $C_{5-20}$-Duftstoffaldehyden und den $C_{5-20}$-Duftstoffketonen besteht;
mit
ii) mindestens einem Derivat der Formel

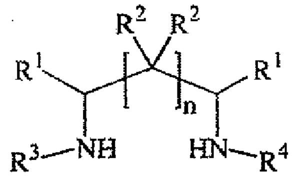

(I)

erhältlich ist, worin:

n eine von 0 bis 3 variierende ganze Zahl darstellt;
$R^1$, unabhängig voneinander, ein Wasserstoffatom, eine optional substituierte Phenylgruppe oder eine optional substituierte $C_{1-18}$-Alkyl- oder -Alkenylgruppe darstellen;
$R^2$, unabhängig voneinander, ein Wasserstoffatom, eine optional substituierte Phenylgruppe, oder eine optional substituierte $C_{1-6}$-Alkyl- oder -Alkenylgruppe darstellen; zwei $R^2$ oder zwei $R^1$ oder ein $R^1$ und ein $R^2$, zusammen genommen, eine $C_{3-5}$-Alkandiyl- oder Alkendiylgruppe bilden können; und
$R^3$ und $R^4$ jeweils eine $C_{1-3}$-Alkylgruppe, substituiert durch eine optional substituierte Phenylgruppe darstellen; $R^3$ und $R^4$ oder $R^3$ und das benachbarte $R^1$, zusammen genommen, eine $C_{2-4}$-Alkandiyl- oder -Alkendiylgruppe bilden können.

**9.** Aminal der Formel

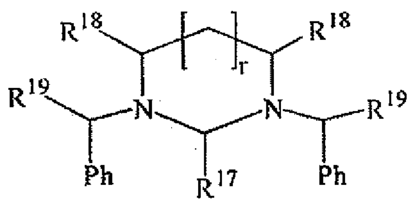

(IV)

worin r 0 oder 1 darstellt;
$R^{19}$, unabhängig voneinander, ein Wasserstoffatom oder eine Methyl- oder Ethylgruppe darstellen;
$R^{18}$, unabhängig voneinander, ein Wasserstoffatom, eine Phenylgruppe, optional substituiert durch eine oder zwei OH- oder $C_1$-$C_4$-Alkyl- oder -Alkoxylgruppe(n), oder eine $C_{1-4}$-Alkylgruppe darstellen; zwei $R^{18}$, zusammen genommen, eine $C_{3-4}$-Alkandiyl- oder -Alkendiylgruppe bilden können;
Ph, unabhängig voneinander, eine Phenylgruppe, optional substituiert durch ein oder zwei $NR^{20}_2$, $(NR^{20}_3)X$, $OR^{20}$, $SO_3M$, $COOR^{20}$ oder $R^{20}$ darstellen, wobei $R^{20}$ eine $C_1$- bis $C_3$- oder $C_4$-Alkylgruppe oder ein Wasserstoffatom darstellt, wobei M ein Wasserstoffatom oder ein Alkalimetallion darstellt, und X ein Halogenatom oder ein Sulfat darstellt; und
$R^{17}$ der Rest eines aktiven Aldehyds $R^{17}CHO$ ist, der ein Molekulargewicht zwischen 80 und 230 g/mol aufweist und ein Duftstoff-, Geschmacksstoff-, Insektenabwehrmittel- oder -lockmittelbestandteil ist, und worin $R^{17}$ Folgendes darstellt: eine $C_6$-$C_{14}$-Alkyl-, -Alkenyl oder Alkadienylgruppe, optional substituiert durch eine OH- oder eine $OR^{15}$ -Gruppe oder eine $C_{1-3}$-Alkyl- oder -Alkenylgruppe, substituiert durch eine Phenylgruppe, die durch eine, zwei oder drei OH-, $R^{15}$ - oder $OR^{15}$-Gruppe(n) optional substituiert ist, wobei $R^{15}$ eine Acetyl- oder eine $C_1$-$C_4$-Alkyl- oder Alkenylgruppe ist;
mit der Maßgabe, dass wenn Ph mit OH- oder OMe-Gruppen substituiert ist und $R^{18}$ und $R^{19}$ Wasserstoffatome sind, $R^{17}$ dann Folgendes darstellt:

- eine $C_7$-$C_{14}$-Alkylgruppe oder eine $C_6$-$C_{14}$-Alkenyl- oder -Alkadienylgruppe,
- eine $C_{1-3}$-Alkylgruppe, substituiert durch eine Phenylgruppe, die durch eine, zwei oder drei OH-, $R^{15}$- oder $OR^{15}$-Gruppe(n) substituiert ist,
- eine durch eine Phenylgruppe substituierte $C_{2-3}$-Alkylgruppe oder
- eine $C_{2-3}$-Alkenylgruppe, substituiert durch eine Phenylgruppe, die durch eine, zwei oder drei OH-, $R^{15}$- oder $OR^{15}$-Gruppe(n) substituiert ist,

$R^{15}$ eine $C_1$-$C_4$-Alkyl- oder -Alkenylgruppe ist;
und mit der Maßgabe, dass 1,2,3-Tribenzylimidazolidin, 1,3-Dibenzyl-2-styrylimidazolidin, 1,3-Dibenzyl-2-hexyl-imidazolidin und 1,3-Bis(4-dimethylaminobenzyl)-2-styryl-imidazolidin ausgeschlossen sind.

**10.** Duftstoffzusammensetzung, umfassend:

i) als Duftstoffbestandteil, ein dynamisches Gemisch wie nach Anspruch 1 definiert;
ii) mindestens einen Bestandteil, der aus der Gruppe ausgewählt ist, die aus einem Duftstoffträger und einer Duftstoffbasis besteht; und
iii) optional mindestens einen Duftstoffzusatz.

**11.** Parfümierter Gegenstand, umfassend:

i) als Duftstoffbestandteil, ein dynamisches Gemisch wie nach Anspruch 1 definiert; und
ii) eine flüssige Verbraucherproduktbasis.

**12.** Parfümierter Gegenstand nach Anspruch 11, **dadurch gekennzeichnet, dass** die flüssige Verbraucherproduktbasis ein Parfüm, Eau de Cologne oder eine After-Shave-Lotion, eine parfümierte Seife, ein Detergens, ein Dusch- oder Bademousse, -Öl oder -Gel, ein Hygieneprodukt oder Haarpflegeprodukt, ein Körperpflegeprodukt, ein Deodorans oder Antiperspirans, ein Lufterfrischer, ein Kosmetikpräparat, ein Gewebeerfrischer, ein Bügelwasser, ein Papier, ein Wischtuch oder ein Bleichmittel, eine Weichmachergrundlage ist.

**13.** Parfümierter Gegenstand nach Anspruch 12, **dadurch gekennzeichnet, dass** das Derivat der Formel (I) N,N'-Dibenzylcyclohexan-1,2-diamin oder N-Benzyl-N-(2-piperidinylmethyl)amin ist.

**14.** Parfümierter Gegenstand, umfassend:

i)

- ein Derivat der Formel (I), wie nach Anspruch 1 definiert, und/oder mindestens ein Aminal, das aus einem Derivat der Formel (I) und einem aktiven Aldehyd oder einem aktiven Keton, wie nach Anspruch 1 definiert, erhältlich ist, und
einen Duftstoff oder eine Duftstoffzusammensetzung mit mindestens einem Duftstoffaldehyd oder Duftstoffketon, der/das ein Molekulargewicht zwischen 80 und 230 g/mol aufweist; oder
- mindestens ein Aminal, das aus einem Derivat der Formel (I) und einem aktiven Aldehyd oder einem aktiven Keton erhältlich ist;
und

ii) eine feste Verbraucherproduktbasis, die zur Verwendung in Anwesenheit von Wasser beabsichtigt ist.

**15.** Parfümierter Gegenstand nach Anspruch 14, **dadurch gekennzeichnet, dass** die feste Verbraucherproduktbasis eine parfümierte Seife, ein Detergens, ein Hygieneprodukt,
ein Körperpflegeprodukt, ein Deodorans oder Antiperspirans, ein Lufterfrischer, ein Kosmetikpräparat, ein Papier, ein Wischtuch oder ein Bleichmittel ist.

**16.** Parfümierter Gegenstand nach Anspruch 15, **dadurch gekennzeichnet, dass** das Derivat der Formel (I) N,N'-Dibenzylcyclohexan-1,2-diamin oder N-Benzyl-N-(2-piperidinylmethyl)amin ist.

## Revendications

**1.** Utilisation, en tant qu'ingrédient parfumant, d'un mélange dynamique, pour la libération contrôlée d'aldéhydes ou de cétones actifs, susceptible d'être obtenu en faisant réagir, dans un milieu contenant de l'eau,

i) au moins un aldéhyde actif ou cétone active ayant une masse moléculaire comprise entre 80 et 230 g/mol et qui est un ingrédient parfumant, aromatisant, insectifuge ou appât pour insectes, choisi dans le groupe constitué des aldéhydes parfumants en $C_{5-20}$ et des cétones parfumantes en $C_{5-20}$;
avec
ii) au moins un dérivé de formule

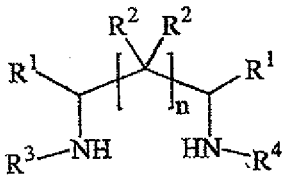

(I)

dans laquelle:

n représente un entier variant de 0 à 3;
les $R^1$ représentent, indépendamment l'un de l'autre, un atome d'hydrogène, un groupe phényle éventuellement substitué, ou un groupe alkyle ou alcényle en $C_{1-18}$ éventuellement substitué;
les $R^2$ représentent, indépendamment l'un de l'autre, un atome d'hydrogène, un groupe phényle éventuellement substitué, ou un groupe alkyle ou alcényle en $C_{1-6}$ éventuellement substitué; deux $R^2$ ou deux $R^1$ ou un $R^1$ et un $R^2$, pris ensemble, peuvent former un groupe alcanediyle ou alcènediyle en $C_{3-5}$; et
$R^3$ et $R^4$ représentent chacun un groupe alkyle en $C_{1-3}$ substitué par un groupe phényle éventuellement substitué; $R^3$ et $R^4$ ou $R^3$ et le $R^1$ adjacent, pris ensemble, peuvent former un groupe alcanediyle ou alcènediyle en $C_{2-4}$.

**2.** Utilisation selon la revendication 1, **caractérisée en ce que** le dérivé de formule (I) est un composé dans lequel:

- le groupe $R^3$ est pris avec le $R^1$ adjacent pour former un groupe alcanediyle ou alcènediyle tel que défini dans la revendication 1; ou
- deux groupes $R^1$, ou deux groupes $R^2$ ou un groupe $R^2$ et un groupe $R^1$, sont pris ensemble pour former un groupe tel que défini dans la revendication 1.

**3.** Utilisation selon la revendication 1, **caractérisée en ce que** le dérivé de formule (I) est un composé de formule

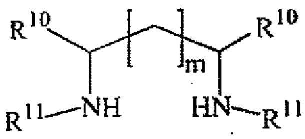

(II)

dans laquelle m représente 0 ou 1;
les $R^{10}$ représentent, indépendamment l'un de l'autre, un atome d'hydrogène, un groupe phényle éventuellement substitué, ou un groupe alkyle en $C_{1-4}$ éventuellement substitué; les deux $R^{10}$, pris ensemble, peuvent former un groupe alcanediyle ou alcènediyle en $C_{3-4}$; et
les $R^{11}$ représentent, indépendamment l'un de l'autre, un groupe alkyle en $C_{1-3}$ substitué par un groupe phényle éventuellement substitué; deux groupes $R^{11}$ ou un groupe $R^{10}$ et un groupe $R^{11}$, pris ensemble, peuvent former un groupe alcanediyle ou alcènediyle en $C_{2-4}$.

**4.** Utilisation selon la revendication 1, **caractérisée en ce que** le dérivé de formule (1) est choisi parmi:

i) $BzNHCH_2(CH_2)_gCH_2NHBz$, où g vaut 1 ou 0 et Bz est un groupe benzyle substitué ou non substitué;
ii) $R^{12}HN-(C_6H_{10})NHR^{12}$ où $R^{12}$ est un groupe Bz tel que défini ci-dessus;
iii) la pipérazine ou le 1,4-diaza-cycloheptane;
iv) $R^{12}HNCHArCHArNHR^{12}$, où $R^{12}$ est un groupe Bz tel que défini ci-dessus et Ar est un groupe phényle; ou
v) $(C_5H_9NH)CH_2NHR^{12}$ où $R^{12}$ est un groupe Bz tel que défini ci-dessus.

**5.** Utilisation selon la revendication 1, **caractérisée en ce que** le dérivé de formule (I) est choisi parmi:

la N,N'-dibenzyléthane-1,2-diamine (N,N'-dibenzyléthylènediamine), la N,N'-dibenzylpropane-1,3-diamine, la N,N'-dibenzylcyclohexane-1,2-diamine, la N,N'-bis[4-(diméthylamino)benzyl]éthane-1,2-diamine, la N,N'-bis [4-(diméthylamino)benzyl]propane-1,3-diamine, la N,N'-bis(4-méthoxybenzyl)éthane-1,2-diamine, la N,N'-bis(4-méthoxybenzyl)propane-1,3-diamine, le 4,4'-[1,2-éthanediyl-bis(iminométhylène)]dibenzoate de diméthyle ou de diéthyle, la N,N'-bis(4-éthylbenzyl)éthane-1,2-diamine, la N,N'-dibenzyl-1,2-diphényléthane-1,2-diamine ou la N-benzyl-N-(2-pipéridinylmëthyl)amine.

**6.** Utilisation selon l'une quelconque des revendications 1 à 5, **caractérisée en ce que** l'aldéhyde actif ou la cétone active est un aldéhyde parfumant ou une cétone parfumante.

**7.** Utilisation selon la revendication 6, **caractérisée en ce que** l'aldéhyde parfumant ou la cétone parfumante est **caractérisé**(e) par une pression de vapeur supérieure à 2,0 Pa.

**8.** Mélange dynamique susceptible d'être obtenu en faisant réagir, dans un milieu contenant de l'eau,

i) au moins deux aldéhydes actifs ou deux cétones actives ayant une masse moléculaire comprise entre 80 et 230 g/mol et qui constituent un ingrédient parfumant, aromatisant, insectifuge ou appât pour insectes, choisis dans le groupe constitué des aldéhydes parfumants en $C_{5-20}$ et des cétones parfumantes en $C_{5-20}$;
avec
ii) au moins un dérivé de formule

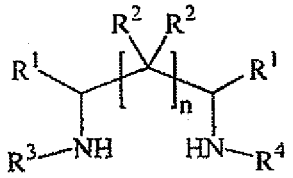

(I)

dans laquelle:

n représente un entier variant de 0 à 3;
les $R^1$ représentent, indépendamment l'un de l'autre, un atome d'hydrogène, un groupe phényle éventuellement substitué, ou un groupe alkyle ou alcényle en $C_{1-18}$ éventuellement substitué;
les $R^2$ représentent, indépendamment l'un de l'autre, un atome d'hydrogène, un groupe phényle éventuellement substitué, ou un groupe alkyle ou alcényle en $C_{1-6}$ éventuellement substitué; deux $R^2$ ou deux $R^1$ ou un $R^1$ et un $R^2$, pris ensemble, peuvent former un groupe alcanediyle ou alcènediyle en $C_{3-5}$; et
$R^3$ et $R^4$ représentent chacun un groupe alkyle en $C_{1-3}$ substitué par un groupe phényle éventuellement substitué; $R^3$ et $R^4$ ou $R^3$ et le $R^1$ adjacent, pris ensemble, peuvent former un groupe alcanediyle ou alcènediyle en $C_{2-4}$.

**9.** Aminal de formule

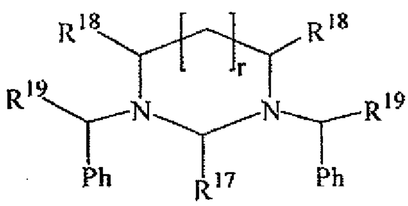

(IV)

dans laquelle r représente 0 ou 1;
les $R^{19}$ représentent, indépendamment l'un de l'autre, un atome d'hydrogène ou un groupe méthyle ou éthyle;
les $R^{18}$ représentent, indépendamment l'un de l'autre, un atome d'hydrogène, un groupe phényle éventuellement substitué par un ou deux groupes OH ou alkyle ou alcoxyle en $C_1$-$C_4$, ou un groupe alkyle en $C_{1-4}$; deux $R^{18}$, pris ensemble, peuvent former un groupe alcanediyle ou alcènediyle en $C_{3-4}$;
les Ph représentent, indépendamment l'un de l'autre, un groupe phényle éventuellement substitué par un ou deux $NR^{20}_2$, $(NR^{20}_3)X$, $OR^{20}$, $SO_3M$, $COOR^{20}$ ou $R^{20}$, $R^{20}$ représentant un groupe alkyle en $C_1$ à $C_3$ ou $C_4$ ou un atome d'hydrogène, M représentant un atome d'hydrogène ou un ion de métal alcalin, et X représentant un atome d'halogène ou un sulfate; et

$R^{17}$ est le résidu d'un aldéhyde actif $R^{17}CHO$ ayant une masse moléculaire comprise entre 80 et 230 g/mol qui est un ingrédient parfumant, aromatisant, insectifuge ou appât pour insectes, et dans lequel $R^{17}$ représente un groupe alkyle, alcényle ou alcadiényle en $C_6$-$C_{14}$ éventuellement substitué par un groupe OH ou $OR^{15}$, ou un groupe alkyle ou alcényle en $C_{1-3}$ substitué par un groupe phényle éventuellement substitué par un, deux ou trois groupes OH, $R^{15}$ ou $OR^{15}$, $R^{15}$ étant un groupe acétyle ou un groupe alkyle ou alcényle en $C_1$-$C_4$;
pour autant que si Ph est substitué par des groupes OH ou OMe et $R^{18}$ et $R^{19}$ sont des atomes d'hydrogène, alors ledit $R^{17}$ représente

- un groupe alkyle en $C_7$-$C_{14}$ ou un groupe alcényle ou alcadiényle en $C_6$-$C_{14}$,
- un groupe alkyle en $C_{1-3}$ substitué par un groupe phényle substitué par un, deux ou trois groupes OH, $R^{15}$ ou $OR^{15}$,
- un groupe alkyle en $C_{2-3}$ substitué par un groupe phényle ou
- un groupe alcényle en $C_{1-3}$ substitué par un groupe phényle substitué par un, deux ou trois groupes OH, $R^{15}$ ou $OR^{15}$,

$R^{15}$ étant un groupe alkyle ou alcényle en $C_1$-$C_4$;
et pour autant que la 1,2,3-tribenzyl-imidazolidine, la 1,3-dibenzyl-2-styryl-imidazolidine, la 1,3-dibenzyl-2-hexyl-imidazolidine et la 1,3-bis(4-dïméthylaminobenzyl)-2-styryl-imidazolidine soient exclues.

**10.** Composition parfumante comprenant:

i) en tant qu'ingrédient parfumant, un mélange dynamique tel que défini dans la revendication 1;
ii) au moins un ingrédient choisi dans le groupe constitué d'un véhicule de parfumerie et d'une base de parfumerie; et
iii) éventuellement, au moins un adjuvant d'usage courant en parfumerie.

**11.** Article parfumé comprenant:

i) en tant qu'ingrédient parfumant, un mélange dynamique tel que défini dans la revendication 1 ; et
ii) une base de produit de consommation liquide.

**12.** Article parfumé selon la revendication 11, **caractérisé en ce que** la base de produit de consommation liquide est un parfum, une eau de Cologne ou une lotion après-rasage, un savon parfumé, un détergent, une mousse, une huile ou un gel de bain ou de douche, un produit d'hygiène ou un produit de soin capillaire, un produit de soin corporel, un déodorant ou un anti-transpirant, un désodorisant d'air ambiant, une préparation cosmétique, une eau de linge, une eau de repassage, un papier, une lingette ou un agent de blanchiment, une base adoucissante.

**13.** Article parfumé selon la revendication 12, **caractérisé en ce que** le dérivé de formule (I) est la N,N'-dibenzylcyclohexane-1,2-diamine ou la N-benzyl-N-(2-pipéridinylméthyl)amine.

**14.** Article parfumé comprenant:

i)

- un dérivé de formule (I), tel que défini dans la revendication 1, et/ou au moins un aminal pouvant être obtenu à partir d'un dérivé de formule (I) et d'un aldéhyde actif ou cétone active, tel que défini dans la revendication 1, et
un parfum ou une composition parfumante contenant au moins un aldéhyde parfumant ou cétone parfumante ayant une masse moléculaire comprise entre 80 et 230 g/mol; ou
- au moins un aminal pouvant être obtenu à partir d'un dérivé de formule (I) et d'un aldéhyde actif ou cétone active;
et

ii) une base de produit de consommation solide destinée à être utilisée en présence d'eau.

**15.** Article parfumé selon la revendication 14, **caractérisé en ce que** la base de produit de consommation solide est un savon parfumé, un détergent, un produit d'hygiène, un produit de soin corporel, un déodorant ou un anti-transpirant, un désodorisant d'air ambiant, une préparation cosmétique, un papier, une lingette ou un agent de blanchiment.

16. Article parfumé selon la revendication 15, **caractérisé en ce que** le dérivé de formule (I) est la N,N'-dibenzylcyclohexane-1,2-diamine ou la N-benzyl-N-(2-pipéridinylméthyl)amine.

Figure 1 : Formation and hydrolysis of 1,3-dibenzyl-2-phenylhexahydropyrimidine followed by $^1$H-NMR spectroscopy in THF-$d_8$/$D_2O$ 2:1 (v/v). The measured values are represented with data points, the lines between the data points correspond to the fitted curve obtained by calculation:

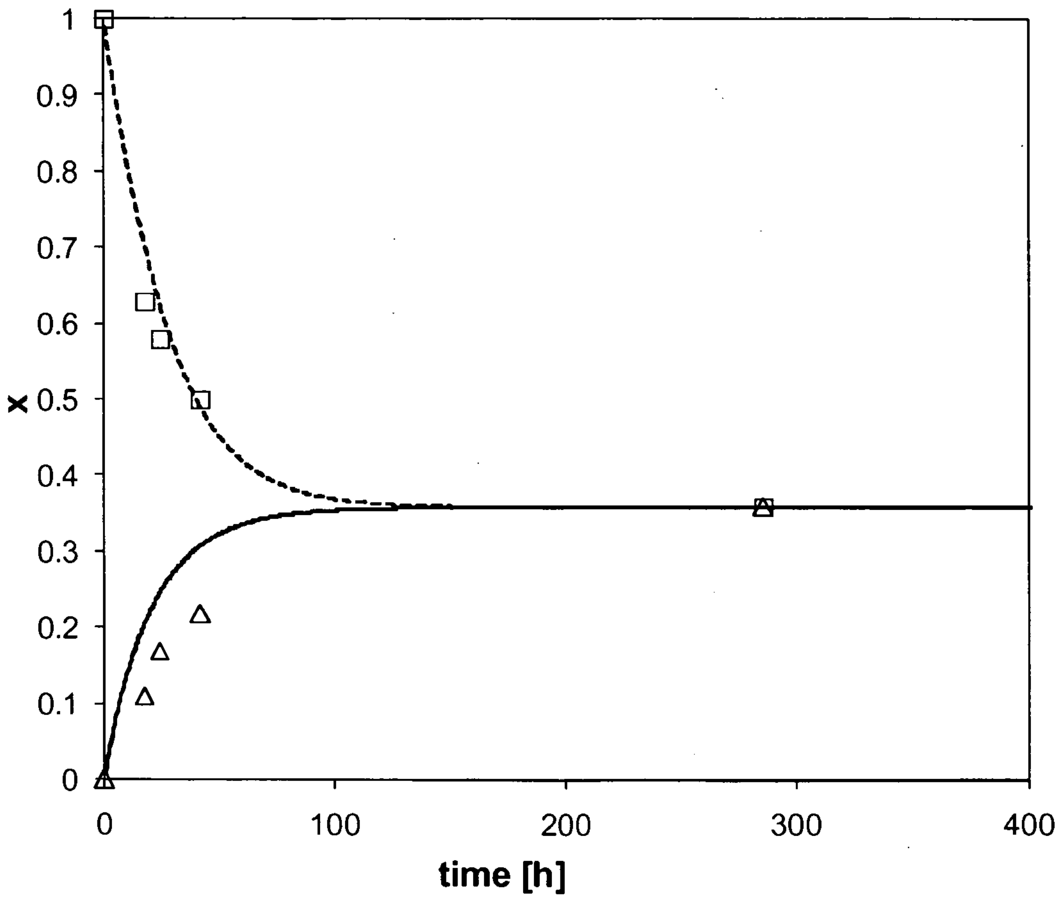

Figure 2 : Comparison of the concentration in the headspace of perfumery aldehydes and ketones in the presence and absence of (1*R*,2*R*)-N,N'-dibenzylcyclohexane-1,2-diamine on dry fabric as obtained by dynamic headspace analysis (presence of the diamine —— solid line; absence of the diamine ---- dashed line):

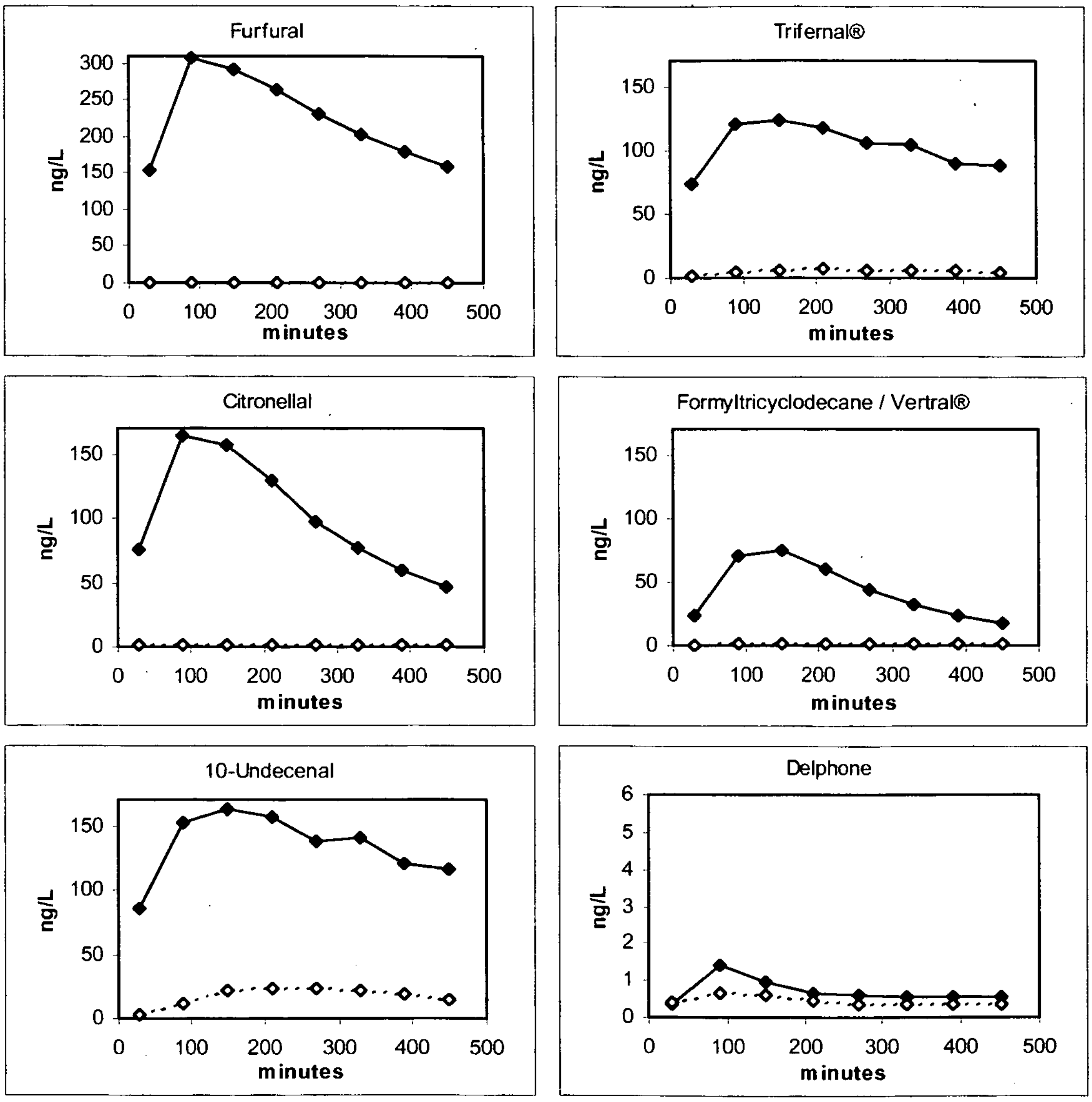

Figure 3 : Comparison of the headspace concentrations measured on dry fabric for a mixture of 18 volatile aldehydes and ketones in the presence (grey bars) or absence (white bars) of N,N'-dimethylethane-1,2-diamine (**A**) and (1*R*,2*R*)-N,N'-dibenzylcyclohexane-1,2-diamine (**D**) (data are represented at the same scale):

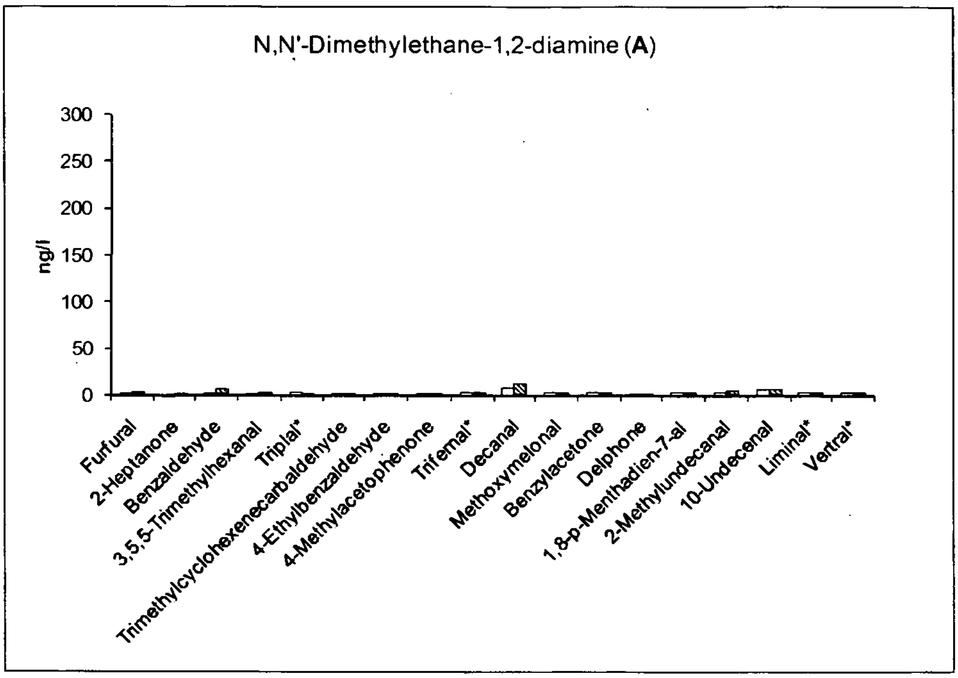


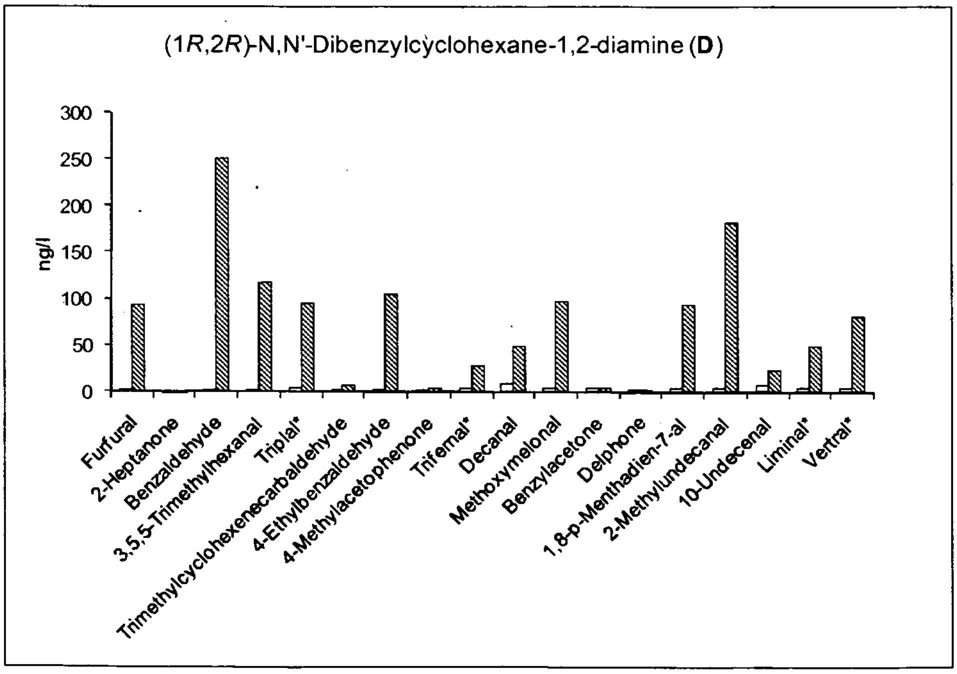


* = registered trademark

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0002991 A
- US 20050239667 A
- WO 0193823 A
- WO 2006016248 A
- DE 102005062175 A1
- WO 2007085991 A


### Non-patent literature cited in the description


- **S. Pawlenko ; S. Lang-Fugmann.** *Houben-Weyl Methoden der organischen Chemie,* 1992, 574
- **H. W. Wanzlick ; W. Lochel.** *Chem. Ber.,* 1953, 1463
- **O. Andrey et al.** *Adv. Synth. Catal.,* 2004, 1147
- **A. Alexakis et al.** *Pure & Appl. Chem.,* 1996, 531
- **S. E. Denmark et al.** *J. Org. Chem.,* 1991, 5063
- **J. Clayden ; L. W. Lai.** *Angew. Chem. Int. Ed.,* 1999, 2556
- **J. Clayden et al.** *Tetrahedron,* 2004, 4399
- **S. Arctander.** *Perfume and Flavor Chemicals,* 1969
- **Ullman's.** Encyclopedia of Industrial Chemistry. 1987, vol. A8, 315-448
- ENCYCLOPEDIA OF INDUSTRIAL CHEMISTRY. 1994, vol. A25, 747-817
- **Flick.** Advanced Cleaning Product Formulations. Noye Publication
- Surfactant Science Series. **Showell.** Powdered Detergents. Marcel Dekker, 1988, vol. 71
- Proceedings of the World Conference on Detergents. AOCS, 1998
- **J. W. Moore ; R. G. Pearson.** Kinetics and Mechanism. John Wiley & Sons, 1981, 284-333